# EUROPEAN PATENT SPECIFICATION

(11) **EP 4 114 313 B1**
(45) Date of publication and mention of the grant of the patent: **01.10.2025**
(21) Application number: 21765014.2
(22) Date of filing: 03.03.2021
(51) Int. Cl.: A61F 2/24

(54) **PROSTHETIC CARDIAC VALVE DEVICES, SYSTEMS**
HERZKLAPPENPROTHESENVORRICHTUNGEN UND -SYSTEME
DISPOSITIFS, SYSTÈMES POUR VALVULES CARDIAQUES PROTHÉTIQUES

(30) Priority: 03.03.2020 US 202062984595 P
(43) Date of publication of application: 11.01.2023
(73) Proprietor: Shifamed Holdings, LLC, Campbell, CA 95008 (US)
(72) Inventor: ARGENTO, Claudio, Campbell, CA 95008 (US); BACKUS, Andrew, Campbell, CA 95008 (US); YANG, Alice, Campbell, CA 95008 (US)
(74) Representative: J A Kemp LLP
(86) International application number: PCT/US2021/020704
(87) International publication number: WO 2021/178560

(56) References cited:
- WO-A1-2019/164516
- US-A- 4 725 274
- US-A1- 2014 379 074
- US-A1- 2016 199 177
- US-A1- 2016 199 177
- US-A1- 2016 228 247
- US-A1- 2017 071 732
- US-A1- 2017 189 177
- US-A1- 2018 250 127
- US-A1- 2020 060 852
- US-B2- 7 534 261

## Description

This application is related to U.S. Provisional Patent Application No. 62/984,595, filed on March 3, 2020, entitled "PROSTHETIC CARDIAC VALVE DEVICES, SYSTEMS, AND METHODS".

This application may also be related to be related to U.S. Patent Application No. 16/546,901, filed on August 21, 2019, entitled "PROSTHETIC CARDIAC VALVE DEVICES, SYSTEMS, AND METHODS," U.S. Patent Application No. 17/170,717, filed on February 8, 2021, entitled "PROSTHETIC CARDIAC VALVE DEVICES, SYSTEMS, AND METHODS," International Patent Application No. PCT/US2019/057082, filed on October 18, 2019, entitled "ADJUSTABLE MEDICAL DEVICE," U.S. Patent Application No. 16/723,537, filed on December 20, 2019, entitled "PROSTHETIC CARDIAC VALVE DEVICES, SYSTEMS, AND METHODS," U.S. Patent Application No. 16/824,576, filed on March 19, 2020, entitled "PROSTHETIC CARDIAC VALVE DEVICES, SYSTEMS, AND METHODS," International Patent Application No. PCT/US2020/027744, file on April 10, 2020, entitled "MINIMAL FRAME PROSTHETIC CARDIAC VALVE DELIVERY DEVICES, SYSTEMS, AND METHODS," and International Patent Application No. PCT/US2020/058413, filed on October 30, 2020, entitled "PROSTHETIC CARDIAC VALVE DEVICES, SYSTEMS, AND METHODS".

Blood flow between heart chambers is regulated by native valves - the mitral valve, the aortic valve, the pulmonary valve, and the tricuspid valve. Each of these valves are passive one-way valves which open and close in response to differential pressures. Patients with valvular disease have abnormal anatomy and/or function of at least one valve. For example, a valve may suffer from insufficiency, also referred to as regurgitation, when the valve does not fully close and allows blood to flow retrograde. As another example, valve stenosis can cause a valve to fail to open properly. Other diseases may also lead to dysfunction of the valves.

While medications may be used to treat valvular disease, in many cases, the defective valve may need to be repaired or replaced at some point during the patient's lifetime. Existing valves and surgical repair and/or replacement procedures, however, may have increased risks, limited lifespans, and/or are highly invasive. Some less-invasive transcatheter options are available, but these options are generally limited to aortic valve procedures, are limited in their patient-to-patient flexibility, and tend to take longer than desired to implant. US 2016/199177 A1 discloses a system including a helical anchor formed as multiple coils adapted to support a heart valve prosthesis with coil portions positioned above and below the heart valve annulus. US 2018/250127 Al discloses a system including a docking device with different coil portions, where the device is configured to assume an axially expanded and compressed state.

Accordingly, a prosthetic valve, surgical repair, and/or surgical replacement procedure that addresses these limitations is desired.

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes. The prosthetic valve and surgical procedures described herein can advantageously provide an efficient and minimally invasive procedure for repair and replacement of heart valves that can accommodate a variety of individual patients. Not necessarily all such aspects or advantages are achieved by any particular embodiment. Thus, various embodiments may be carried out in a manner that achieves or optimizes one advantage or group of advantages taught herein without necessarily achieving other aspects or advantages as may also be taught or suggested herein.

The present disclosure generally relates to treating a diseased native valve in a patient and more particularly relates to prosthetic heart valves.

The present disclosure relates to prosthetic cardiac devices, and in some embodiments, prosthetic heart valves such as catheter-based mitral valves.

Additionally, existing valve repair/replacement procedures are often complicated and time-consuming. Currently available procedures often require the placement of more than one component - for example, a prosthetic valve and a mechanism to anchor it to the native anatomy. Such procedures generally utilize multiple delivery catheters to carry the various components and delivery of each component separately to the valve, which can be time-consuming (particularly if components are delivered sequential), complicated, and/or dangerous. For example, some devices provide rotational anchoring elements to capture the native anatomy such as the chordae tendineae in order to reduce delivery time. However, such anchoring elements, often by design, capture and pull the chordae along during their rotation, which can torque or otherwise stress and damage the chordae during deployment of the anchor elements, resulting in the need for additional medical interventions for the patient. Additionally, such anchoring elements may require extrusion from a low-profile (e.g., elongated) delivery configuration to an expanded configuration at or near the native valve. In at least some instances, it may be beneficial to provide an anchor with varying stiffness between an undeployed configuration and a deployed configuration. When in the undeployed configuration, increased flexibility may be desired in order to reduce strains and forces within the delivery device. In the deployed configuration, increased stiffness may be desired in order to ensure adequate rigidity of the anchor for encircling the one or more structures of the native valve and the frame structure.

The present disclosure generally relates to treating a diseased native valve in a patient and more particularly relates to anchoring elements with variable stiffness and flexibility.

In a first aspect, a system for treating a diseased native valve in a patient is provided. The system comprises a) a frame structure having an unexpanded configuration and an expanded configuration and b) an anchor comprising a free end and having a flat spiral shape, wherein the anchor comprises housing, a lumen disposed within the housing, and a plurality of spiral bands translatably disposed within the lumen. The frame structure is configured to be actuated from the unexpanded configuration to the expanded configuration adjacent a native valve in a patient. The anchor is configured to anchor the frame structure to the native valve when the frame structure is in the expanded configuration adjacent the native valve.

The plurality of spiral bands are slidably disposed within the lumen relative to one another.

In some embodiments, the anchor may be configured to be fully advanced from an atrial side of the native valve into a ventricle of the heart. Optionally, at least a portion of the anchor may be configured to reside within a subvalvular plane of the heart when it anchors the frame structure to the native valve. In some embodiments, the anchor may be configured to reside entirely within the subvalvular plane of the heart when it anchors the frame structure to the native valve.

In some embodiments, the anchor may comprise a delivery configuration and a deployed configuration. The anchor may comprise the flat spiral shape in the deployed configuration. Alternatively, or in combination, the anchor may comprise an elongated shape in the delivery configuration. In some embodiments, the anchor may be configured to be actuated from the delivery configuration to the deployed configuration adjacent a native valve in a patient.

In some embodiments, the anchor may comprise one or more locking mechanisms configured to maintain the anchor in the deployed configuration. The one or more locking mechanisms may comprise a frictional band, a polymer coating, or one or more key and one or more key hole features. Alternatively, or in combination, the one or more locking mechanisms may comprise a pin on a first spiral band of the plurality of spiral bands and a correspondingly-shaped hole on a second spiral band of the plurality of spiral bands. Alternatively, or in combination, the one or more locking mechanisms may comprise a plurality of slots on the plurality of spiral bands and a correspondingly-shaped pin. Alternatively, or in combination, the one or more locking mechanisms may comprise a first feature disposed on a first loop of the anchor and a correspondingly-shaped second feature disposed on a second loop of the anchor, the first and second features configured to nest with one another when the anchor is in the deployed configuration.

In some embodiments, the deployed configuration may comprise an intermediate deployed configuration and a fully deployed configuration. The anchor may be configured to be actuated from the intermediate deployed configuration to the fully deployed configuration adjacent a native valve in a patient. An outer perimeter of the flat spiral shape may be the same in the intermediate deployed configuration and the fully deployed configuration. The anchor may comprise a first loop, a second loop, and one or more locking mechanisms. The one or more locking mechanisms may be configured to couple the first loop to the second loop when the anchor is in the fully deployed configuration.

In some embodiments, the system may further comprise a delivery device. The delivery device may comprise an outer sheath. The anchor may be disposed in a lumen of the outer sheath and maintained in the delivery configuration by radial constriction from the outer sheath. Advancement of the anchor out of the lumen of the outer sheath may actuate the anchor into the deployed configuration. In some embodiments, the delivery device may further comprise an inner shaft and the inner shaft may be disposed within the lumen of the outer sheath. Optionally, a proximal portion of the frame structure or a proximal end of the anchor may be coupled to a distal portion of the inner shaft. In some embodiments, advancement of the inner shaft towards an opening of the lumen of the outer sheath may advance the anchor out of the lumen of the outer shaft and actuate the anchor into the deployed configuration. In some embodiments, the frame structure may be maintained in the unexpanded configuration by radial constriction from the outer sheath and advancement of the inner shaft out of the lumen of the outer sheath may actuate the frame structure into the expanded configuration. In some embodiments, the delivery device may further comprise guidewire disposed within a lumen of the inner shaft. In some embodiments, the outer sheath may be steerable. In some embodiments, the anchor may be configured to wrap at least partially around the inner shaft in the deployed configuration. Alternatively, or in combination, a central point of the flat spiral shape may be co-axial with a longitudinal axis of the inner shaft when the anchor is in the deployed configuration. In some embodiments, the frame structure may be detachably coupled to the delivery device in the unexpanded configuration during delivery to the native valve. In some embodiments, expansion of the frame structure to the expanded configuration may detach the frame structure from the delivery device.

In some embodiments, a proximal end of the anchor may be coupled to a distal end of the frame structure.

In some embodiments, the flat spiral shape may comprise a one or more loops. The one or more loops comprise at least 360 degrees of rotation. In some embodiments, the one or more loops may comprise about 540 degrees of rotation.

In some embodiments, the flat spiral shape may comprise a plurality of loops. The anchor may comprise one or more spaces between the plurality of loops. The plurality of loops may spiral radially outward from a central point.

In some embodiments, the anchor may comprise a super-elastic material. For example, the anchor may comprise nitinol.

In some embodiments, the housing may comprise nitinol. Alternatively, or in combination, the housing may comprise polyethylene terephthalate.

In some embodiments, the frame structure may comprise an expandable stent.

In some embodiments, the expanded configuration may be a generally tubular expanded shape.

In some embodiments, the frame structure may comprise an expanded outer periphery in the expanded configuration and a compressed outer periphery when subject to an external radial force in the unexpanded configuration. The compressed outer periphery may be smaller in diameter than the expanded outer periphery.

In some embodiments, the frame structure may be balloon-expandable.

In some embodiments, the frame structure may be self-expanding.

In some embodiments, the free end may comprise an atraumatic tip. For example, the free end may comprise a ball tip.

In some embodiments, the free end may be configured for piercing tissue.

In some embodiments, the free end may be bent distally.

In some embodiments, the free end may be bent proximally.

In some embodiments, the free end may be "L"-shaped.

In some embodiments, the anchor may have a circular, tubular, hollow, square, elongated, or triangular cross-section.

In some embodiments, the frame structure may be configured for expanding within the native valve of the patient.

In some embodiments, the unexpanded configuration may be sized and dimensioned for percutaneous insertion and the expanded configuration may be sized and dimensioned for implantation in the native valve of the patient.

In some embodiments, the frame structure may comprise a first and second opposite ends, the first end extending above a native valve and the second end extending below the native valve when the frame structure is anchored to the native valve.

In some embodiments, the frame structure may sit below the native valve when the frame structure is anchored to the native valve.

In some embodiments, the system may further comprise a valve segment within the frame structure comprising a biocompatible one-way valve. In some embodiments, at least a portion of the valve segment may be positioned within at least a portion of the frame structure. In some embodiments, the valve segment may comprise at least one leaflet having an inner layer and an outer layer, and the frame structure may be attached to the outer layer at one or more ends of the frame structure. In some embodiments, the valve segment may comprise a plurality of leaflets.

In another aspect, method for treating a diseased native valve in a patient is provided. The method comprises a) advancing a frame structure and an anchor having a flat spiral shape from a first side of a native valve to a second side of the native valve, wherein the anchor comprises housing, a lumen disposed within the housing, and a plurality of spiral bands translatably disposed within the lumen; b) deploying the anchor adjacent the native valve; c) expanding the frame structure within the native valve from an unexpanded configuration to an expanded configuration; and d) anchoring the frame structure in the expanded configuration to the native valve with the anchor.

In some embodiments, deploying the anchor may comprise deploying the anchor from a delivery configuration to a deployed configuration.

In some embodiments, the anchor may be configured to wrap at least partially around a delivery device in the deployed configuration.

In some embodiments, the anchor may be configured to wrap at least partially around the frame structure in the deployed configuration.

In some embodiments, a central point of the flat spiral shape may be co-axial with a longitudinal axis of a delivery device when the anchor is in the deployed configuration.

In some embodiments, deploying the anchor may comprise deploying the anchor within a subvalvular plane of the heart.

In some embodiments, the method may further comprise moving the deployed anchor into a subvalvular plane of the heart.

In some embodiments, deploying the anchor may comprise actuating the anchor from the delivery configuration to the deployed configuration. Optionally, the method may further comprise maintaining the anchor in the deployed configuration with one or more locking mechanisms. In some embodiments, actuating the anchor may comprise releasing the frame structure from radial constriction by a delivery device. In some embodiments, deploying the anchor may comprise actuating the anchor from the delivery configuration to the deployed configuration on the second side of the native valve. In some embodiments, deploying the anchor comprises actuating the anchor from the delivery configuration to the deployed configuration on the first side of the native valve.

In some embodiments, the deploying the anchor may comprise actuating the anchor from the delivery configuration to the deployed configuration on the first side of the native valve and advancing the anchor may comprise advancing the anchor in the deployed configuration through the native valve to the second side of the native valve. Advancing the anchor may comprise pushing the anchor through the native valve. Advancing the anchor may further comprise rotating the anchor through the native valve.

In some embodiments, the deployed configuration may comprise an intermediate deployed configuration and a fully deployed configuration. Actuating the anchor from the delivery configuration to the deployed configuration may comprise actuating the anchor from the delivery configuration to the intermediate deployed configuration. The method may further comprise actuating the anchor from the intermediate deployed configuration to the fully deployed configuration adjacent a native valve in a patient. In some embodiments, an outer perimeter of the flat spiral shape may be the same in the intermediate deployed configuration and the fully deployed configuration. In some embodiments, the anchor may comprise a first loop, a second loop, and one or more locking mechanisms, and the method may further comprise coupling the first loop to the second loop with the one or more locking mechanisms to maintain the anchor in the fully deployed configuration.

In some embodiments, deploying the anchor may comprise positioning the anchor such that it is located only on the second side of the native valve.

In some embodiments, the frame structure may be detachably coupled to a distal end of a delivery device. Advancing the frame structure may comprise advancing the distal end of the delivery device from the first side of the native valve to the second side of the native valve. The method may further comprise releasing the frame structure from the distal end of the delivery device. In some embodiments, the method may further comprise steering the distal end of the delivery device such that the distal end of the delivery device points towards the first side of the native valve. In some embodiments, expanding the frame structure and releasing the frame structure may occur simultaneously. In some embodiments, the anchor may be detachably coupled to the distal end of the delivery device and advancing the frame structure and advancing the anchor may occur simultaneously.

In some embodiments, the anchor may be detachably coupled to a distal end of a delivery device. Advancing the anchor may comprise advancing the distal end of the delivery device from the first side of the native valve to the second side of the native valve. The method may further comprise releasing the anchor from the distal end of the delivery device. The method may further comprise steering the distal end of the delivery device such that the distal end of the delivery device points towards the first side of the native valve. The frame structure may be detachably coupled to a distal end of a second delivery device and advancing the frame structure and advancing the anchor may occur independently of one another.

In some embodiments, the frame structure may a first and second opposite ends and wherein expanding the frame structure comprises expanding the frame structure such that the first end extends above the first side of the native valve and the second end extends below second side of the native valve.

In some embodiments, expanding the frame structure may comprise expanding at least a portion the frame structure within at least a portion of the deployed anchor to anchor the frame structure to the native valve.

In some embodiments, the frame structure may be balloon-expandable and expanding the frame structure may comprise inflating a balloon disposed within the frame structure, where inflation of the balloon causes expansion of the frame structure.

In some embodiments, the frame structure may be self-expanding and expanding the frame structure may comprise releasing the frame structure from radial constriction by a delivery device.

In some embodiments, the anchor may comprise a free end and anchoring may comprise rotating the free end of the anchor around one or more structures on the second side of the native valve. The method may further comprise counter-rotating the free end of the anchor after rotating the free end around the one or more structures. The one or more structures comprise one or more valve leaflets of the native valve. Alternatively, or in combination, the one or more structures may comprise one or more chordae of the left ventricle.

In some embodiments, the free end may comprise an atraumatic tip. For example, the free end may comprise a ball tip.

In some embodiments, the free end may be configured for piercing tissue.

In some embodiments, the free end may be bent distally.

In some embodiments, the free end may be bent proximally.

In some embodiments, the free end may be "L"-shaped.

In some embodiments, the anchor may have a circular, tubular, hollow, square, elongated, or triangular cross-section.

In some embodiments, the frame structure may comprise a valve segment therewithin comprising a biocompatible one-way valve.

In some embodiments, the native valve may be in a heart of a patient. The method may further comprise transseptally inserting a distal end of a delivery device detachably coupled to the frame structure or to the anchor into a left atrium of the heart. In some embodiments, the native valve may comprise a mitral valve, the first side of the native valve may comprise a left atrium, and the second side of the native valve may comprise a left ventricle.

These and other embodiments are described in further detail in the following description related to the appended drawing figures.

### BRIEF DESCRIPTION OF THE DRAWINGS

The novel features of the present disclosure are set forth with particularity in the appended claims. A better understanding of the features and advantages of the present disclosure will be obtained by reference to the following detailed description that sets forth illustrative embodiments, in which the principles of the present disclosure are utilized, and the accompanying drawings of which:
FIG. 1 shows a side view of a valve prosthesis system comprising a frame structure and an anchor loaded on a delivery device with the anchor in a delivery (e.g., elongated) configuration, in accordance with embodiments.
FIG. 2 shows a side view of the valve prosthesis system of FIG. 1 with the anchor in a deployed configuration, in accordance with embodiments.
FIGS. 3A-3AE show sequential views of a method of implanting a valve prosthesis using a delivery device, in accordance with embodiments.
FIGS. 4-6 show several views of a valve prosthesis with an anchor comprising a spiral band, in accordance with embodiments.
FIGS. 7-9 show several views of the anchor of FIGS. 4-6 loaded on a delivery device, in accordance with embodiments.
FIGS. 10-11 show deployment of the anchor of FIGS. 4-6 from an unexpanded, undeployed configuration (FIG. 10) to an expanded, deployed configuration (FIG. 11) in accordance with embodiments.
FIGS. 12-13 show an optional locking mechanism for a spiral band anchor, in accordance with embodiments.
FIG. 14 shows another optional locking mechanism for a spiral band anchor, in accordance with embodiments.
FIGS. 15-16 show an optional tip orientation for a spiral band anchor, in accordance with embodiments.
FIGS. 17-18 show another optional tip orientation for a spiral band anchor, in accordance with embodiments.
FIGS. 19-21 show various optional configurations for a spiral band anchor, in accordance with embodiments.
FIGS. 22-24 show several views of an anchor comprising a tapered spiral band, in accordance with embodiments.
FIGS. 25-28 show several views of another anchor comprising a tapered spiral band, in accordance with embodiments.
FIGS. 29-32 show several views of an optional locking mechanism for a spiral band anchor, in accordance with embodiments.
FIGS. 33-37 show several views of a multi-ply anchor, according to the present invention.
FIGS. 38A-39 show several views of a multi-ply anchor comprising an optional stiffening mechanism, according to the present invention.
FIGS. 40-43 show several views of a multi-ply anchor comprising an optional stiffening mechanism, according to the present invention.

### DETAILED DESCRIPTION

In the following detailed description, reference is made to the accompanying figures, which form a part hereof. In the figures, similar symbols typically identify similar components, unless context dictates otherwise. The illustrative embodiments described in the detailed description, figures, and claims are not meant to be limiting. Other embodiments may be utilized, and other changes may be made, without departing from the scope of the subject matter presented herein. It will be readily understood that the aspects of the present disclosure, as generally described herein, and illustrated in the figures, can be arranged, substituted, combined, separated, and designed in a wide variety of different configurations, all of which are explicitly contemplated herein.

The scope of the claims appended hereto is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments, however, the order of description should not be construed to imply that these operations are order dependent. Additionally, the structures, systems, and/or devices described herein may be embodied as integrated components or as separate components.

For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

The present disclosure is described in relation to deployment of systems, devices, or methods for treatment of a diseased native valve of the heart, for example a mitral valve, aortic valve, or tricuspid. However, one of skill in the art will appreciate that this is not intended to be limiting and the devices and methods disclosed herein may be used in other anatomical areas and in other surgical procedures.

For convenience in explanation and accurate definition in the appended claims, the terms "up" or "upper", "down" or "lower", "inside" and "outside" are used to describe features of the present disclosure with reference to the positions of such features as displayed in the figures.

In many respects the modifications of the various figures resemble those of preceding modifications and the same reference numerals followed by subscripts "a", "b", "c", and "d" designate corresponding parts. It will be understood by one of ordinary skill in the art that modifications of corresponding parts of the various figures are interchangeable with one another between embodiments to arrive at multiple combinations with multiple modified parts.

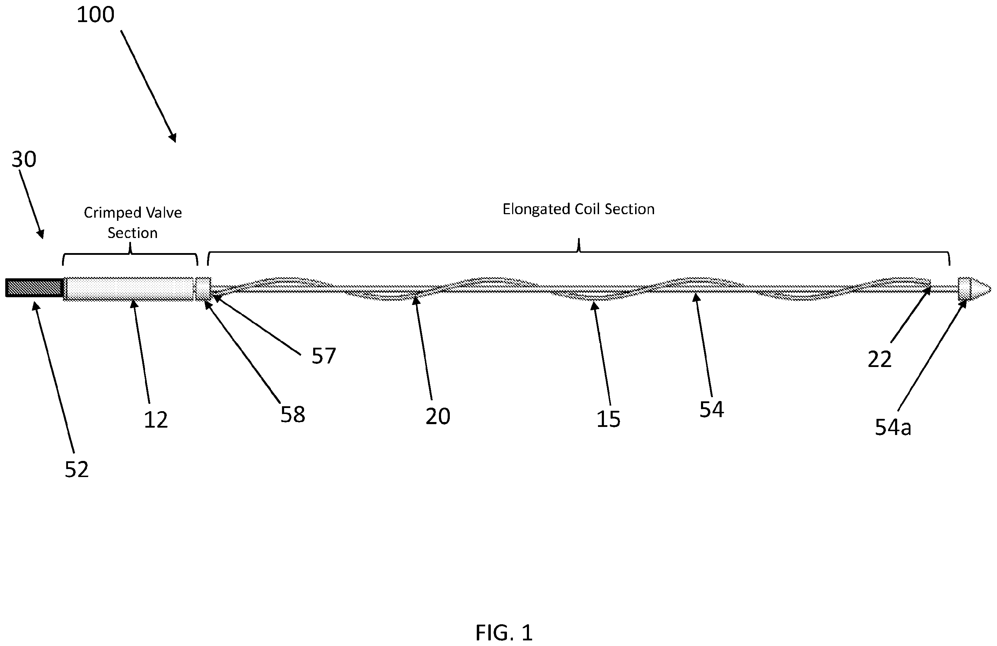

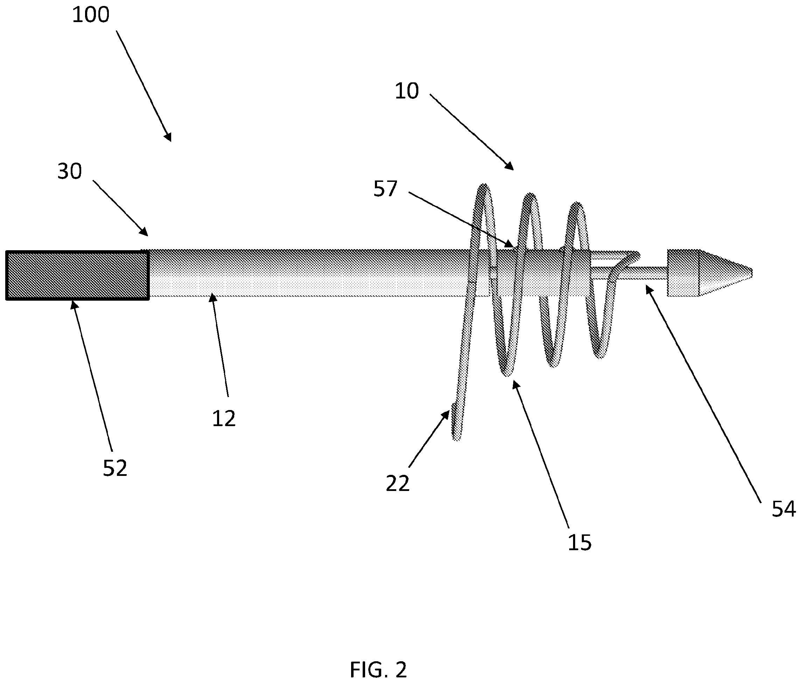

FIG. 1 shows a side view of a valve prosthesis system 100 comprising a frame structure 12 and an anchor 15 loaded on a distal end of a delivery device 30 with the anchor 15 in an elongated, low-profile delivery configuration. FIG. 2 shows a side view of a valve prosthesis system 100 of FIG. 1 comprising a valve prosthesis 10 loaded on a distal end of a delivery device 30 with the anchor 15 in a deployed expanded configuration.

The valve prosthesis 10 may thus have an unexpanded delivery configuration (for example, a compressed configuration as described herein) and an expanded configuration. The valve prosthesis 10 in the expanded configuration may have a generally tubular expanded shape. In some embodiments, the unexpanded configuration may be sized and dimensioned for percutaneous insertion, and the expanded configuration may be sized and dimensioned for implantation in the native valve of the patient. The valve prosthesis 10 may be configured to be fully advanced from a first side of a native valve in a patient (e.g., an atrial side) to a second side of the native valve (e.g., into a ventricle of the heart) and anchor to the native valve when the valve prosthesis 10 is in the expanded configuration adjacent the native valve. It will be understood that the valve prosthesis 10 may be any valve prosthesis described herein or understood based on the teachings herein to one of ordinary skill in the art.

In various embodiments, the unexpanded configuration of the valve prosthesis 10 may be 18Fr or smaller. In various embodiments, the unexpanded configuration may be 20Fr or smaller. In various embodiments, the unexpanded configuration may be 22Fr or smaller. In various embodiments, the unexpanded configuration may be 24Fr or smaller. In various embodiments, the unexpanded configuration may be 26Fr or smaller. In various embodiments, the unexpanded configuration may be 27Fr or smaller. In various embodiments, the unexpanded configuration may be 28Fr or smaller. In various embodiments, the unexpanded configuration may be 29Fr or smaller. In various embodiments, the unexpanded configuration may be 30Fr or smaller. In various embodiments, the unexpanded configuration may be sized and dimensioned for transseptal access. In various embodiments, the unexpanded configuration may be sized and dimensioned for femoral access.

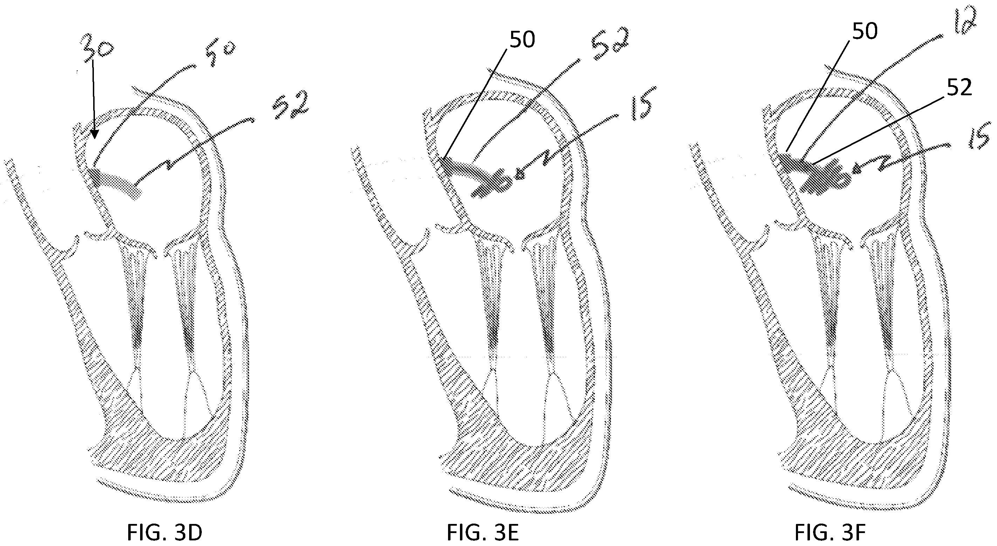

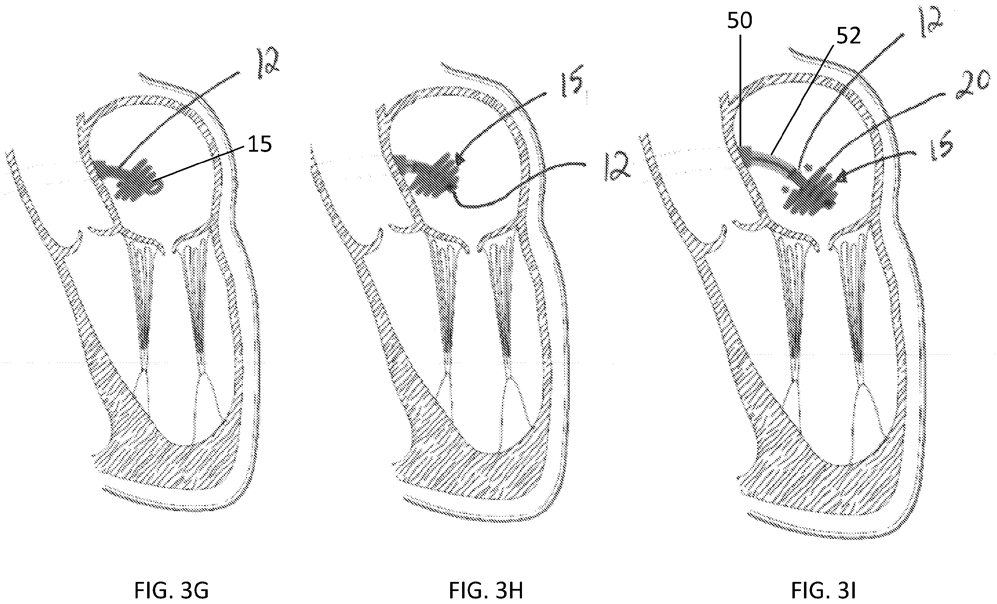

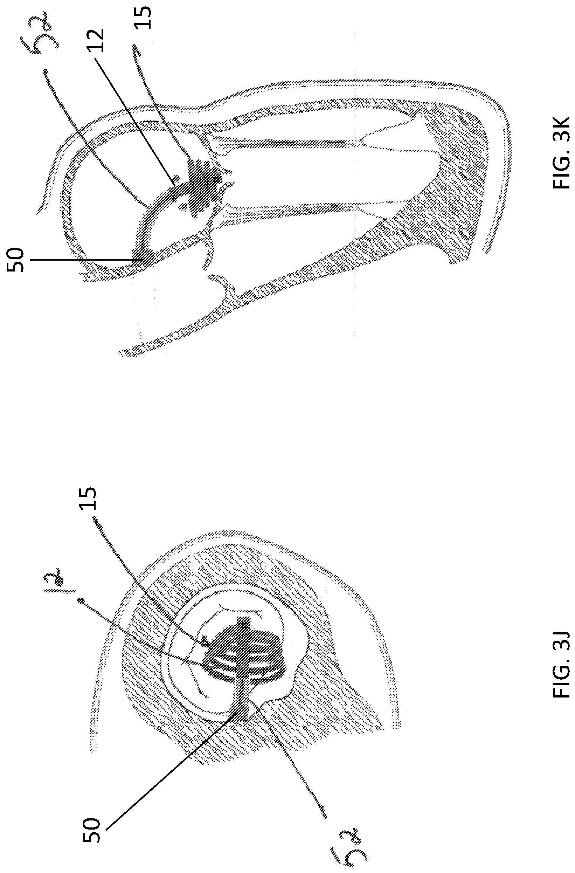

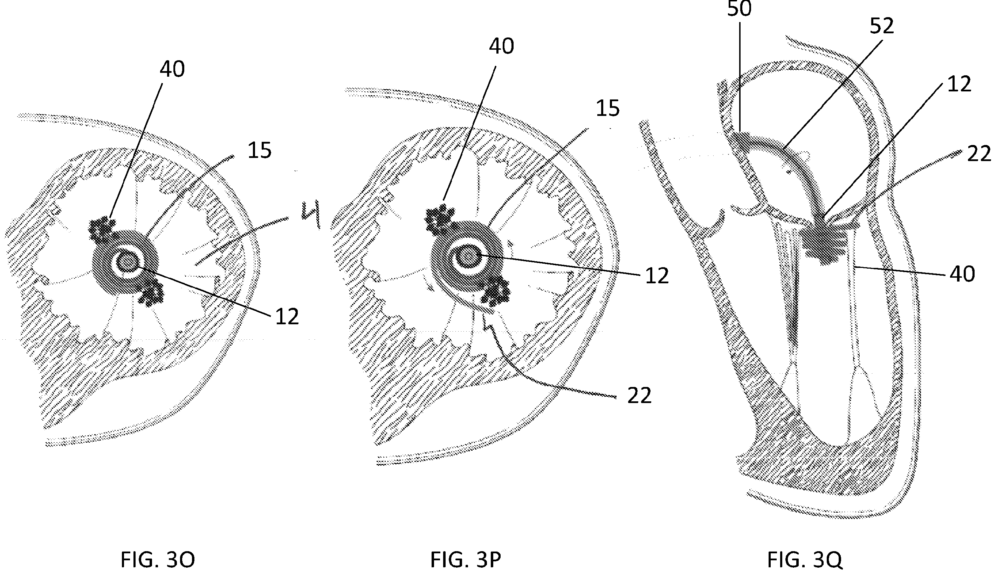

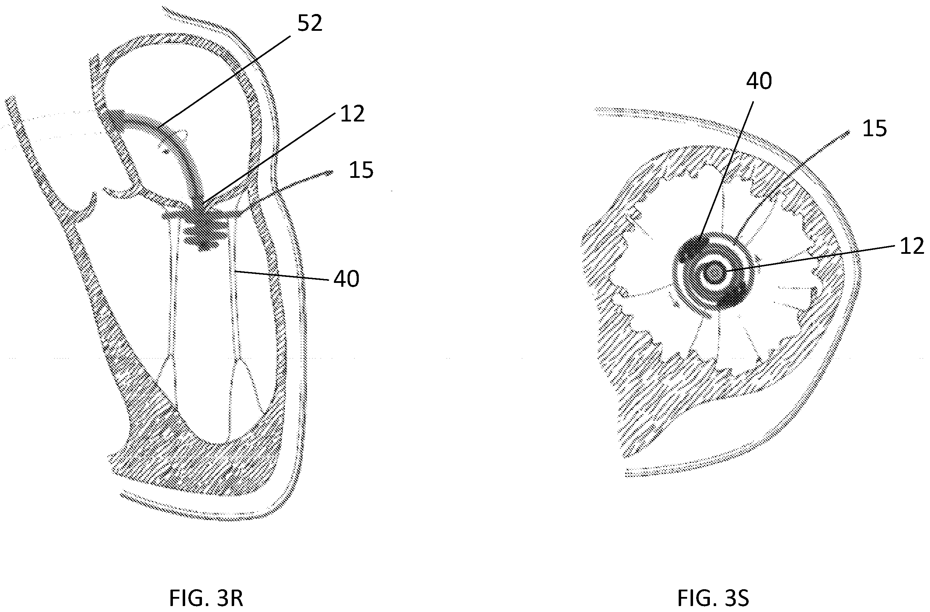

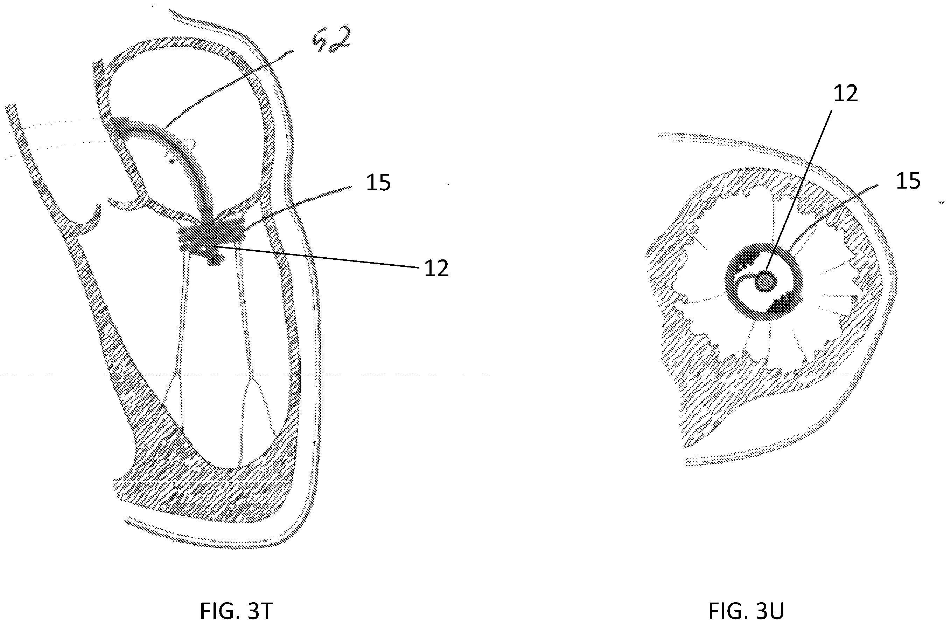

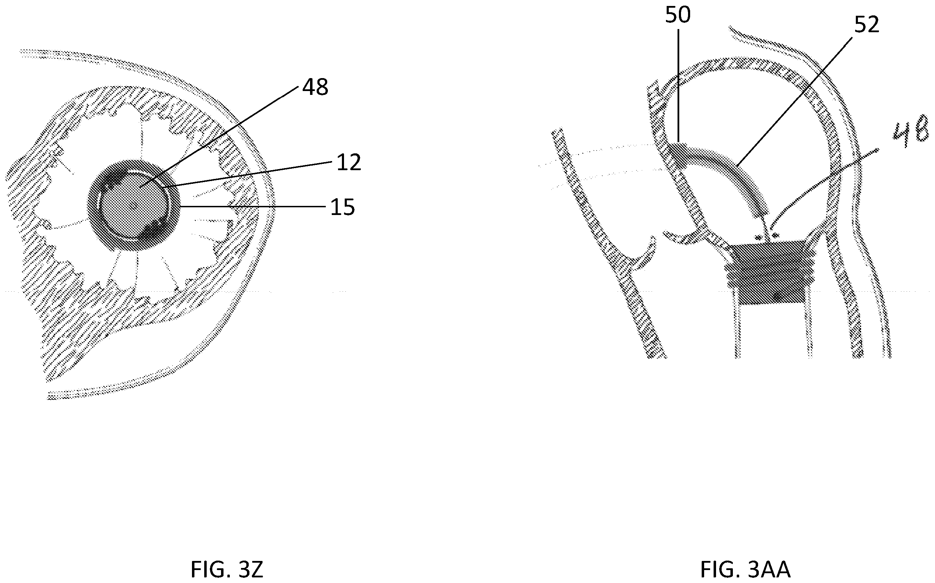

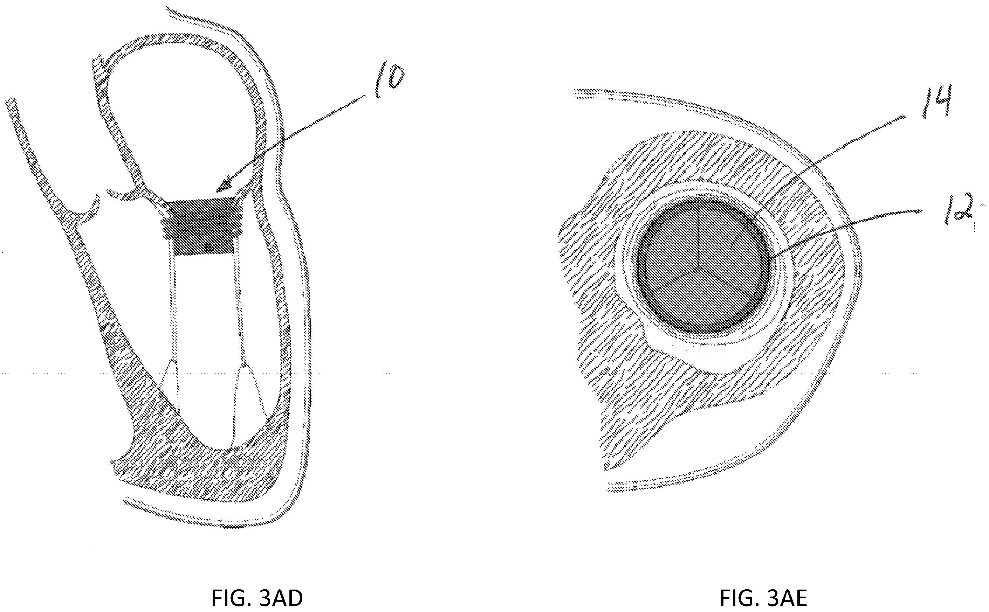

The valve prosthesis 10 may comprise a valve segment (for example, valve segment 14 shown in FIG. 3AE) disposed therein. Valve segment is used somewhat interchangeably with prosthetic valve leaflet and generally refers to the prosthetic leaflets and frame. As used herein, "prosthetic valve" may refer to all manner of prosthetic and artificial replacement valves including tissue (biological valves), tissue-engineered valves, polymer valves (e.g., biodegradable polymer valves), and even certain mechanical valves. The valve segment can be similar to existing transcatheter valves. The valve segment can be similar to existing surgical tissue valves and/or mechanical valves. At least a portion of the valve segment may be positioned within at least a portion of the valve prosthesis 10, for example with a frame structure of the valve prosthesis. The valve segment may include leaflets formed of multi-layered materials for preferential function. The valve segment may comprise at least one leaflet having an inner layer and an outer layer. The valve segment may be attached to a valve structure which is in turn connected to the valve prosthesis 10. The valve structure may be connected to the valve prosthesis 10 before or after the valve prosthesis 10 has been deployed adjacent a native valve. The valve segment may be attached directly to the valve prosthesis 10. The valve prosthesis 10 may be attached to a leaflet, for example an outer layer of a leaflet, at one or more ends of the valve prosthesis 10. The valve prosthesis 10 may be attached to a leaflet, for example an outer layer of a leaflet, at one or more intermediate portions of the valve prosthesis 10. The valve segment may comprise a plurality of leaflets. The valve segment may comprise a biocompatible one-way valve. Flow in one direction may cause the leaflet(s) to deflect open, and flow in the opposite direction may cause the leaflet(s) to close.

In some embodiments, frame structure 12 is a closed frame such that blood flow is forced through valve segment therein. One or more skirts and/or seals may help force blood through valve segment.

The valve prosthesis may be substantially similar to any of the valve prostheses described in U.S. Patent Application No. 16/546,901, filed on August 21, 2019, entitled "PROSTHETIC CARDIAC VALVE DEVICES, SYSTEMS, AND METHODS," U.S. Patent Application No. 17/170,717, filed on February 8, 2021, entitled "PROSTHETIC CARDIAC VALVE DEVICES, SYSTEMS, AND METHODS," International Patent Application No. PCT/US2019/057082, filed on October 18, 2019, entitled "ADJUSTABLE MEDICAL DEVICE," U.S. Patent Application No. 16/723,537, filed on December 20, 2019, entitled "PROSTHETIC CARDIAC VALVE DEVICES, SYSTEMS, AND METHODS," U.S. Patent Application No. 16/824,576, filed on March 19, 2020, entitled "PROSTHETIC CARDIAC VALVE DEVICES, SYSTEMS, AND METHODS," International Patent Application No. PCT/US2020/027744, file on April 10, 2020, entitled "MINIMAL FRAME PROSTHETIC CARDIAC VALVE DELIVERY DEVICES, SYSTEMS, AND METHODS," and International Patent Application No. PCT/US2020/058413, filed on October 30, 2020, entitled "PROSTHETIC CARDIAC VALVE DEVICES, SYSTEMS, AND METHODS.

The delivery device 30 may comprise an outer sheath (e.g., an outer catheter, not shown), an inner shaft 52 (e.g., a delivery tube) disposed within a lumen of the outer sheath, and an optional guidewire 54 disposed within a lumen of the inner shaft 52. The guidewire 54 may optionally comprise a nosecone 54a to facilitate guidance of the guidewire to the native valve. A proximal end 57 of the anchor 15 may be detachably coupled to the inner shaft 52 during delivery to the native valve as described herein. The outer sheath may be steerable.

The valve prosthesis 10 may be operably coupled to the delivery device 30 as described herein. In some embodiments, at least a portion of the valve prosthesis 10 may be directly coupled to the inner shaft 52. Alternatively, or in combination, at least a portion of the valve prosthesis 10 may be indirectly coupled to the inner shaft 52. For example, at least a portion of the valve prosthesis 10 may be coupled to a torque hub or other connector, which may be coupled to the inner shaft 52, such as the torque hub described in International Patent Application No. PCT/US2020/027744, file on April 10, 2020, entitled "MINIMAL FRAME PROSTHETIC CARDIAC VALVE DELIVERY DEVICES, SYSTEMS, AND METHODS.

Additional description for the delivery device and other similar delivery devices usable in the embodiments described herein may be found in U.S. Patent Application No. 16/546,901, filed on August 21, 2019, entitled "PROSTHETIC CARDIAC VALVE DEVICES, SYSTEMS, AND METHODS," U.S. Patent Application No. 17/170,717, filed on February 8, 2021, entitled "PROSTHETIC CARDIAC VALVE DEVICES, SYSTEMS, AND METHODS," International Patent Application No. PCT/US2019/057082, filed on October 18, 2019, entitled "ADJUSTABLE MEDICAL DEVICE," U.S. Patent Application No. 16/723,537, filed on December 20, 2019, entitled "PROSTHETIC CARDIAC VALVE DEVICES, SYSTEMS, AND METHODS," U.S. Patent Application No. 16/824,576, filed on March 19, 2020, entitled "PROSTHETIC CARDIAC VALVE DEVICES, SYSTEMS, AND METHODS," International Patent Application No. PCT/US2020/027744, file on April 10, 2020, entitled "MINIMAL FRAME PROSTHETIC CARDIAC VALVE DELIVERY DEVICES, SYSTEMS, AND METHODS," and International Patent Application No. PCT/US2020/058413, filed on October 30, 2020, entitled "PROSTHETIC CARDIAC VALVE DEVICES, SYSTEMS, AND METHODS,".

At least a portion of the valve prosthesis 10 may be expanded within the native valve. For example, the valve prosthesis 10 may be deployed such that it captures one or more structures therein, for example one or more chordae tendineae and/or one or more valve leaflets. Expansion of the valve prosthesis 10, or a portion thereof, may compress the captured structures therein to anchor the valve prosthesis 10 in place.

The valve prosthesis 10 may comprise first and second opposite ends, the first end extending above a native valve and the second end extending below the native valve when the valve prosthesis 10 is anchored to the native valve. Alternatively, the valve prosthesis 10 may be configured to sit entirely below the native valve (e.g., in the ventricle) when the valve prosthesis 10 is anchored to the native valve.

In some embodiments, the valve prosthesis 10 may comprise an expanded outer periphery in the expanded configuration and a compressed outer periphery when subject to an external radial force in the unexpanded configuration. The compressed outer periphery may be smaller in diameter than the expanded outer periphery.

In some embodiments, at least a portion of the valve prosthesis 10 may be actuated from the unexpanded configuration to the expanded configuration on a first side of the native valve prior to being advanced to a second side of the native valve. For example, the valve prosthesis 10 may be expanded in a left atrium of a heart prior to being advanced to a left ventricle of the heart as described herein.

Alternatively, or in combination, at least a portion of the valve prosthesis 10 may be actuated from the unexpanded configuration to the expanded configuration on a second side of the native valve after being advanced to the second side from a first side of the native valve. For example, valve prosthesis 10 may be advanced from a left atrium of a heart prior to being expanded in a left ventricle of the heart.

The valve prosthesis 10 may be detachably coupled to the delivery device 30 in the unexpanded configuration during delivery to the native valve. Expansion of the valve prosthesis 10 to the expanded configuration may detach (i.e. release) the valve prosthesis 10 from the delivery device 30.

The valve prosthesis 10 may be balloon-expandable, self-expanding, or otherwise expansible, as will be understood to one of ordinary skill in the art from the description herein.

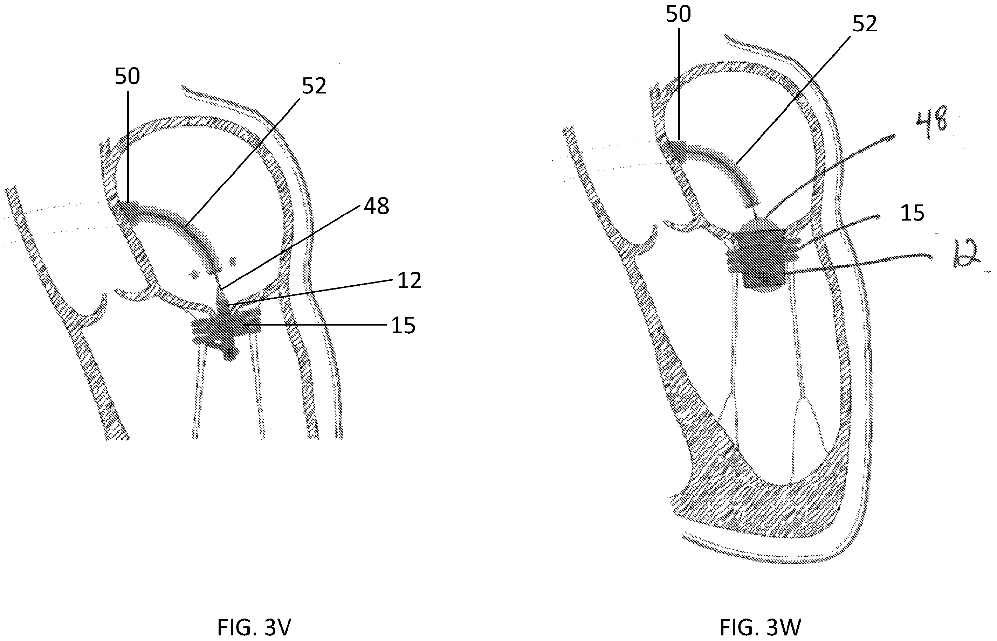

For example, the delivery system 30 may comprise an inflatable balloon (for example, as shown in FIGS. 3V-3AA) disposed within the valve prosthesis 10. Inflation of the balloon may cause expansion of the valve prosthesis 10.

Alternatively, or in combination, the valve prosthesis 10 may be self-expandable. The valve prosthesis 10 may be maintained in the unexpanded configuration by radial constriction from the outer sheath of the delivery device 30 when disposed in a lumen of the outer sheath. Advancement of the inner shaft 52 out of the lumen of the outer sheath may actuate the valve prosthesis 10 into the expanded configuration. Stated another way, retraction of the outer sheath away from the valve prosthesis 10 may actuate the valve prosthesis 10 into the expanded configuration.

The valve prosthesis 10 may, for example, comprise a frame structure 12 and an anchor 15. The anchor 15 may be directly coupled to the frame structure 12, for example at a proximal or distal end thereof. Alternatively, or in combination, the anchor 15 may be detachably coupled to the delivery device 30 prior to deployment at the native valve. For example, a proximal end 57 of the anchor 15 may be detachably coupled to the inner shaft 52 during delivery to the native valve. The proximal end 57 may be configured to remain engaged with the inner shaft 52 after being actuated from the elongated configuration to the deployed configuration adjacent the native valve. Alternatively, or in combination, a proximal end 57 of the anchor 15 may be coupled to a distal end of the frame structure 12 or a proximal end of the frame structure 12.

The frame structure 12 may have an unexpanded configuration (for example, as shown in FIGS. 1, 2 and 3F-3V), for example when the valve prosthesis 10 is in its unexpanded configuration, and an expanded configuration (for example, as shown in FIGS. 3W-3AE), for example when the valve prosthesis 10 is in its expanded configuration. The expanded configuration of the frame structure 12 may have a generally tubular expanded shape. The frame structure 12 may be configured for expanding within the native valve of the patient. In some embodiments, the unexpanded configuration may be sized and dimensioned for percutaneous insertion, and the expanded configuration may be sized and dimensioned for implantation in the native valve of the patient. The frame structure 12 may be configured to remain in its unexpanded configuration while the anchor 15 is in the deployed configuration (e.g., during delivery).

The frame structure 12 may comprise a first and second opposite ends, the first end extending above a native valve and the second end extending below the native valve when the frame structure 12m is anchored to the native valve. Alternatively, the frame structure 12 may be configured to sit entirely below the native valve when the frame structure 12 is anchored to the native valve.

In some embodiments, the frame structure 12 may comprise an expanded outer periphery in the expanded configuration and a compressed outer periphery when subject to an external radial force in the unexpanded configuration. The compressed outer periphery may be smaller in diameter than the expanded outer periphery.

The frame structure 12 may be balloon-expandable, self-expanding, or otherwise expansible as will be understood by one of ordinary skill in the art. The frame structure 12 may, for example, comprise an expandable stent.

The delivery system 30 may comprise an inflatable balloon (for example as shown in FIGS. 3V-3AA) disposed within the frame structure 12 and inflation of the balloon may cause expansion of the frame structure 12 as described herein.

Alternatively, or in combination, the frame structure 12 may be self-expanding and may be maintained in the unexpanded configuration by radial constriction from the outer sheath of the delivery device 30. Advancement of the inner shaft 52 out of the lumen of the outer sheath may actuate the frame structure 12 into the expanded configuration.

The frame structure 12 and/or anchor 15 may be operably coupled to the delivery device 30 as described herein. In some embodiments, at least a portion of the frame structure 12 and/or anchor 15 may be directly coupled to the inner shaft 52. For example, a proximal portion of the frame structure 12 and/or a proximal portion of the anchor 15 may be coupled to a distal portion of the inner shaft 52. Alternatively, or in combination, at least a portion of the frame structure 12 and/or anchor 15 may be indirectly coupled to the inner shaft 52.

The frame structure 12 may be detachably coupled to the delivery device 30 in the unexpanded configuration during delivery to the native valve. Expansion of the frame structure 12 to the expanded configuration may detach the frame structure 12 from the delivery device 30.

At least a portion the frame structure 12 may be expanded within at least a portion of the deployed anchor 15 to anchor the frame structure 12 to the native valve. For example, the anchor 15 may be deployed such that it captures one or more structures therein, for example one or more chordae tendineae and/or one or more valve leaflets. Expansion of the frame structure 12, or a portion thereof, within the anchor 15 may compress the captured structures between the frame structure 12 and the anchor 15 to anchor the frame structure 12 in place.

The frame structure 12 may be configured like a stent. The frame structure 12 may, for example, comprise a scaffold in a diamond pattern formed from a shape memory material (e.g., NiTi). One of ordinary skill in the art will appreciate that many other structures, materials, and configurations may be employed for the frame structure 12. For example, the frame structure 12 may be formed of a polymer of sufficient elasticity. The frame structure 12 may be formed of a combination of metal and polymer, such as metal (e.g., shape memory material) covered in polymer. The frame structure 12 may include a variety of patterns besides diamond shapes.

The frame structure 12 may comprise a valve segment disposed therein as described herein. The valve segment may be attached to a valve structure which is in turn connected to the frame structure 12. The valve structure may be connected to the frame structure 12 before or after the frame structure 12 has been deployed adjacent a native valve. The valve segment may be attached directly to the frame structure 12. The frame structure 12 may be attached to a leaflet, for example an outer layer of a leaflet, at one or more ends of the frame structure 12. The frame structure 12 may be attached to a leaflet, for example an outer layer of a leaflet, at one or more intermediate portions of the frame structure 12.

Additional description for the frame structure and other similar frame structures usable in the embodiments described herein may be found in U.S. Patent Application No. 16/546,901, filed on August 21, 2019, entitled "PROSTHETIC CARDIAC VALVE DEVICES, SYSTEMS, AND METHODS," U.S. Patent Application No. 17/170,717, filed on February 8, 2021, entitled "PROSTHETIC CARDIAC VALVE DEVICES, SYSTEMS, AND METHODS," International Patent Application No. PCT/US2019/057082, filed on October 18, 2019, entitled "ADJUSTABLE MEDICAL DEVICE," U.S. Patent Application No. 16/723,537, filed on December 20, 2019, entitled "PROSTHETIC CARDIAC VALVE DEVICES, SYSTEMS, AND METHODS," U.S. Patent Application No. 16/824,576, filed on March 19, 2020, entitled "PROSTHETIC CARDIAC VALVE DEVICES, SYSTEMS, AND METHODS," International Patent Application No. PCT/US2020/027744, file on April 10, 2020, entitled "MINIMAL FRAME PROSTHETIC CARDIAC VALVE DELIVERY DEVICES, SYSTEMS, AND METHODS," and International Patent Application No. PCT/US2020/058413, filed on October 30, 2020, entitled "PROSTHETIC CARDIAC VALVE DEVICES, SYSTEMS, AND METHODS," .

The anchor 15 may comprise a band or wire 20 having a free (e.g., distal) end 22. The other end (e.g., proximal end) of the anchor 15 may be coupled to the top (proximal end) or bottom (distal end) of the frame structure 12 as described herein. Alternatively, or in combination, the other (proximal) end of the anchor 15 may not be attached to the frame structure 12 as described herein. The anchor 15 may be configured to wrap at least partially around the frame structure 12 in the deployed configuration.

The anchor 15 may be configured to be fully advanced from a first side of a native valve in a patient (e.g., an atrial side) to a second side of the native valve (e.g., into a ventricle of the heart) and anchor the frame structure 12 to the native valve when the frame structure 12 is in the expanded configuration adjacent the native valve.

The anchor 15 may comprise a delivery (e.g., elongated) configuration (shown in FIG. 1) and a deployed configuration (shown in FIG. 2). The frame structure 12 may be configured to remain in its unexpanded configuration while the anchor 15 is in the deployed configuration. In various embodiments, the anchor 15 may be self-expanding and may move to the deployed configuration as it is removed from the delivery sheath. In various embodiments, the anchor 15 may be configured to self-assemble when it is deployed in the heart cavity (e.g., left ventricle or atrium). The anchor 15 may be configured to be actuated from the delivery configuration to the deployed configuration adjacent the native valve using any method or mechanism understood by one of ordinary skill in the art from the description herein. For example, retraction of the guidewire 54 into the lumen of the inner shaft 52 may actuate the anchor 15 into the deployed configuration. Alternatively, or in combination, the anchor 15 may be maintained in the delivery configuration by radial constriction from the outer sheath. Advancement of the inner shaft 52 out of the lumen of the outer sheath may actuate the anchor 15 into the deployed configuration as described herein.

Although referred to as an anchor, one will appreciate that anchor 15 does not require performing an anchor function in the traditional sense. As will be described in more detail below, the anchor 15 can guide the valve prosthesis 10 into a desired position within a native valve. The anchor 15 may also mitigate against undesired entanglement and disturbances to the chordae tendineae and valve leaflets of the mitral valve.

In some embodiments, the anchor 15 may be configured to wrap at least partially around a distal portion of the delivery device 30, for example around the inner shaft 52, in the deployed configuration.

In some embodiments, the anchor 15 may be actuated from the delivery configuration to the deployed configuration on a first side of the native valve prior to being advanced to a second side of the native valve. For example, the anchor 15 may be deployed in a left atrium of a heart prior to being advanced to a left ventricle of the heart as described herein.

Alternatively, the anchor 15 may be actuated from the delivery configuration to the deployed configuration on a second side of the native valve after being advanced to the second side from a first side of the native valve. For example, anchor 15 may be advanced from a left atrium of a heart prior to being deployed in a left ventricle of the heart.

The anchor 15 may be detachably coupled to a proximal or distal portion of the frame structure 12 as described herein. Alternatively, or in combination, the anchor 15 may be detachably coupled to the delivery device 30 in the delivery configuration during delivery to the native valve. For example, the proximal end 57 of the anchor 15 may be detachably coupled to the inner shaft 52 of the delivery device 30 by radial constriction from the outer sheath. Retraction of the outer sheath away from the proximal end 57 of the anchor 15 (or, similarly, extrusion of the distal end of the anchor 15 out of an opening in the outer shaft) may detach the anchor 15 from the delivery device 30. Alternatively, or in combination, the proximal end 57 of the anchor 15 may be detachably coupled to the inner shaft 32 of the delivery device 30 by an attachment element 58. Alternatively, or in combination, the proximal end 57 of the anchor 15 may be detachably coupled to the inner shaft 52 of the delivery device 30 by a weak adhesive. Alternatively, or in combination, the proximal end 57 of the anchor 15 may be detachably coupled to the inner shaft 52 by radial constriction from the inner shaft 52.

The anchor 15 may be configured to rotate when the inner shaft 52 is rotated. The anchor 15 may be configured to rotate independently of the inner shaft 52. Rotation of the anchor 15 may aid in advancement of the anchor 15 to the second side of the native valve. Alternatively, or in combination, rotation of the anchor 15, for example a wire 20 comprising a free end 22, may aid in capture of one or more structures on the second side of the native valve by the free end 22 as described herein. By capturing one or more structures on the second side of the native valve, the anchor 15 may maintain its position relative to the native valve and provide an anchor point for the frame structure 12 when in the expanded configuration.

The wire 20 of the anchor 15 may comprise a curved wire in the deployed configuration, for example a coiled wire or band, a helical wire or band, or a spiral wire or band as described herein. The wire 20 may be shaped to encircle chordae and/or leaflets of a diseased native valve. In various embodiments, the wire or band 20 may have a curved shape in the deployed configuration. In various embodiments, the wire or band 20 may be elongated-rather than curved-in the delivery configuration. For example, the wire or band 20 may be elongated into a straight shape within the delivery device 30. In various embodiments, a portion of the wire or band 20 may have a curved encircling shape. In various embodiments, a substantial portion of the wire or band 20 may have a curved encircling shape. In various embodiments, the wire or band 20 may be formed as a flat curve (in the deployed configuration) whereby the loops generally are positioned within the same plane (the plane being perpendicular to a longitudinal axis). In various embodiments, the wire or band 20 may be formed as a three-dimensional curve (in the deployed configuration) whereby the loops generally are positioned out of plane with one another.

The wire or band 20 may comprise a helical wire in the deployed configuration. As used herein, a helix or helical shape may comprise a curve for which the tangent at any point along the curve makes a constant angle with a fixed line (e.g., a central axis). The curve may turn around an axis at a constant or continuously varying distance while moving parallel to the axis. In some embodiments, the wire or band 20 may comprise one or more helical portions as described herein.

In various embodiments, wire 20 has varying stiffness along its length. The wire 20 may have two or more segments of differing stiffness and/or the stiffness may transition over its length. In various embodiments, wire 20 is attached to frame 12 at multiple points such that free end 22 is relatively flexible and the wire 20 is more rigid along portions where it is attached to the frame structure 12.

In various embodiments, free end 22 extends radially outward from frame structure 12, and in particular the remainder of wire 20. As will be described below, the free end 22 is configured to encircle a larger radius than the main coils of the wire 20. When the main coils of wire 20 have a generally curved shape (e.g., spiral, helical, tubular, frustoconoical, etc.), the free end 22 may extend radially outward from the curved shape. For example, when the main coils of wire 20 have a generally spiral shape, the free end 22 may extend radially outward from the spiral shape. When the main coils of wire 20 have a generally tubular shape, the free end 22 may extend radially outward from the tubular shape. When the main coils of wire 20 have a generally helical shape, the free end 22 may extend radially outward from the helical shape. When the main coils of wire 20 have a generally frustoconical shape, the free end 22 may extend radially outward from the frustoconical shape. When the main coils of wire 20 have a generally cylindrical shape, the free end 22 may extend radially outward from the cylindrical shape. The free end 22 may be configured to encircle a larger radius than the main coils of the helical wire 20. The larger diameter can facilitate capturing of the valve leaflets and/or chordae tendineae within the sweep of the free end 22 when rotated as described herein.

In various embodiments, the wire or band 20 may have a generally helical shape in the deployed configuration. In various embodiments, the wire or band 20 may be elongated-rather than helix-shaped-in the delivery configuration. For example, the wire or band 20 may be elongated into a straight shape within the delivery device. In various embodiments, a portion of the wire or band 20 may have a helical shape. In various embodiments, a substantial portion of the wire or band 20 may have a helical shape. In various embodiments, the helical wire or band 20 may be formed as a three-dimensional helix (in the deployed configuration) whereby the loops generally are positioned around the same axis (for example, a longitudinal axis of the delivery device 30).

Optionally, the anchor 15 may comprise a first portion comprising the helical wire 20 and another portion. Alternatively, or in combination, the anchor 15 may comprise a plurality of helical wires 20. For example, the anchor 15 may comprise at least two helical wires 20 having the same or different diameters. Alternatively, or in combination, the anchor 15 may comprise at least two helical wires 20 having the same or different winding pitches.

The wire 20 may comprise a spiral wire or band in the deployed configuration. As used herein, a spiral or spiral shape may comprise a curve which emanates from a point (e.g., a central point) having a continuously increasing or decreasing distance from the point. The spiral or spiral shape may be two-dimensional (e.g., planar) or three-dimensional. In some embodiments, the wire or band 20 may comprise one or more spiral portions as described herein.

In various embodiments, the anchor and/or core wire may have a spiral-shaped deployed configuration. In various embodiments, spiral refers to a shape with windings about a central axis. The spiral may be continuous. The windings may gradually widen (or tighten) along the length. The spiral may be formed in a flat plane perpendicular to the central axis. In various embodiments, the anchor and/or core wire may have a deployed configuration that is not formed in a flat plane, or in other words the deployed shape is formed in a three-dimensional and/or nondegenerate space. In various embodiments, the anchor and/or core wire may have a conical-shaped deployed configuration including, but not limited to, tubular, conical, frustoconical, and/or helical shapes.

In various embodiments, the anchor 15 may comprise a flat spiral shape. Loops of the flat spiral shaped anchor may be generally positioned within the same plane (the plane being perpendicular to a longitudinal axis of a delivery device) as described herein.

The free end 22 of the spiral band or wire 20 may extend radially outward from the frame 12, and in particular from the remainder of the spiral band or wire 20. The other end of the spiral band or wire 20 may be coupled to the top or bottom of the frame structure 12 as described herein. Alternatively, or in combination, the other end of the spiral band or wire 20 may not be attached to the frame structure 12 as described herein. The free end 22 of the spiral band or wire 20 may facilitate capturing of the valve leaflets and/or chordal tendineae within the sweep of the free end during rotation as described herein. During rotation of the spiral band or wire 20, the leaflets and/or chordae tendineae may be captured by the free end 22 and trapped between the valve frame structure 12 and an interior surface of the spiral band or wire 20.

In various embodiments, the wire or band 20 may have a generally spiral shape in the deployed configuration. In various embodiments, the wire or band 20 may be elongated-rather than spiral-shaped-in the delivery configuration. For example, the wire or band 20 may be elongated into a straight shape within the delivery device. In various embodiments, a portion of the wire or band 20 may have a spiral shape. In various embodiments, a substantial portion of the wire or band 20 may have a spiral shape. In various embodiments, the spiral wire or band 20 may be formed as a flat spiral (in the deployed configuration) whereby the loops generally are positioned within the same plane (the plane being perpendicular to a longitudinal axis).

Optionally, the anchor 15 may comprise a first portion comprising the spiral wire 20 and another portion. Alternatively, or in combination, the anchor 15 may comprise a plurality of spiral wires 20. For example, the anchor 15 may comprise at least two spiral wires 20 having the same or different diameters. Alternatively, or in combination, the anchor 15 may comprise at least two spiral wires 20 having the same or different winding pitches.

The wire 20 may comprise one or more loops. For example, the wire 20 may comprise a plurality of loops, which may increase the radial strength of the anchor by increasing friction and addition structural support. The one or more loops of the wire 20 may be substantially cylindrical around a central axis of the wire 20, for example along an axis which is coaxial with a longitudinal axis of a delivery device 30. The one or more loops of the wire 20 may be substantially helical around a central axis or helical axis of the wire 20, for example along an axis which is coaxial with a longitudinal axis of a delivery device. The one or more loops of the wire 20 may spiral radially outward from a central point or central axis of the wire 20, for example along an axis which is coaxial with a longitudinal axis of a delivery device 30 such that the wire 20 lies approximately along a plane perpendicular to the longitudinal axis of the delivery device 30.

The one or more loops (also referred to herein as coils or turns) of the wire or band 20 may comprise a curved shape that bends around back towards its origin (for example, an arc, ellipsoid, circle, or the like). In some embodiments, a loop may comprise a curved shape that bends back towards its origin but does not cross itself, making a rotation within a range of about 180 degrees to about 360 degrees. For example, a loop may comprise a curve or an arc having a central angle within a range of about 180 degrees to about 360 degrees. In at least some embodiments, the one or more loops may comprise an arc. In some embodiments, a loop may comprise a shape that bends back towards and crosses itself, making at least a 360 degree rotation. In at least some embodiments, the one or more loops may comprise a 360 degree rotation (for example, a circle). In some embodiments, the one or more loops may comprise a 360 degree to 720 degree rotation (for example, a loop crossing itself once and rotating further towards a second crossing and formation of a second 360 loop).

The one or more loops may comprise any number of loops desired, for example, one, two, three, four, five, six, seven, eight, nine, or ten loops. The one or more loops may comprise a rotation within a range of about 180 degrees to about 3600 degrees. The one or more loops may comprise a rotation within a range bounded by any of the following values: 180 degrees, 270 degrees, 360 degrees, 450 degrees, 540 degrees, 630 degrees, 720 degrees, 810 degrees, 900 degrees, 990 degrees, 1080 degrees, 1170 degrees, 1260 degrees, 1350 degrees, 1440 degrees, 1530 degrees, 1620 degrees, 1710 degrees, 1800 degrees, 1890 degrees, 1980 degrees, 2070 degrees, 2160 degrees, 2250 degrees, 2340 degrees, 2430 degrees, 2520 degrees, 2610 degrees, 2700 degrees, 2790 degrees, 2880 degrees, 2970 degrees, 3060 degrees, 3150 degrees, 3240 degrees, 3330 degrees, 3420 degrees, 3510 degrees, or 3600 degrees.

Interaction of the frame structure 12 with the one or more loops of the anchor 15 may create opposing forces therebetween that provide mechanical leverage for anchoring the frame structure 12 to the one or more anatomical structures. In some embodiments, the one or more loops may comprise at least 360 degrees of rotation when deployed such that the loops wrap around one another and provide additional mechanical leverage against the frame structure 12 in order to facilitate anchoring of the frame structure 12 as described herein. Additional loops or partial loops may provide additional mechanical strength and/or leverage.

In some embodiments, the one or more loops of the wire or band 20 may comprise one or more spaces therebetween. The spaces may facilitate movement of the captured tissue (e.g. chordae and/or leaflets) from the free end 22 to the center of the spiral structure during rotation of the anchor 15 as described herein.

The wire 20 may be configured to wrap at least partially around the frame structure 12 in the deployed configuration.

In some embodiments, the wire 20 may be configured to wrap at least partially around a distal portion of the delivery device 30, for example around the inner shaft, in the deployed configuration.

The free end 22 of the wire 20 may be sized and dimensioned for insertion through the native valve, for example through tissue at or near a commissure of the native valve or through the valve opening itself. In some embodiments, the free end 22 of the wire 20 may comprise an atraumatic tip to avoid reduce risk of injury to the native valve tissue and leaflets. For example, the free end may comprise a blunt end, a ball tip, a curved tip (e.g., J-tip or pigtail), or other atraumatic shapes. Alternatively, the free end 22 of the wire 20 may be configured for piercing tissue.

In various embodiments, the free end 22 is separate from and extends outward from the main coils of the anchor 15. In various embodiments, the main body coils of the anchor 15 circumscribe an area (in the case of a spiral coil) or a volume (in the case of a helical coil) having a diameter, and the free end 22 extends to a radius greater than the diameter of the anchor 15. In various embodiments, the free end 22 extends to a radius substantially greater than the diameter of the anchor 15. In various embodiments, the free end 22 is configured to circumscribe a larger diameter than the anchor 15. In various embodiments, the free end 22 is configured to circumscribe all of the chordae tendineae of the native valve to be treated.

In various embodiments, the free end 22 may be shaped and configured to reduce the risk of counter-rotation. For example, the tip 22 may have a curled end to cause the free end 22 to snag chordae if it is rotated in a direction opposite the anchoring rotation.

The free end 22 of the wire or band 20 may optionally be rotated around one or more structures on the second side of the native valve such that the one or more structures (e.g., chordae, leaflets, or annulus) are pulled radially inwards towards the longitudinal axis of the anchor 15 and/or towards the longitudinal axis of the delivery device 30. The wire or band 20 and/or free end 22 may be configured such that minimal torque is applied to the one or more structures. Alternatively, or in combination, the wire or band 20 and/or free end 22 may be configured such that the one or more structures are not rotated, or are minimally rotated, during rotation of the wire or band 20. For example, the wire or band 20 may comprise one or more spaces between loops of the curved wire or band which facilitate radially-inward movement of the captured tissue (e.g. chordae and/or leaflets) from the free end 22 to the center of the curved structure with little or no torque and/or rotation of the structures during rotation of the wire or band 20 as described herein. Alternatively, or in combination, the wire or band 20 may be configured such that, when fully deployed none of the structures reside between the loops of the curved encircling shape. Instead, the one or more structures may sit radially inward of the loops in order to facilitate capture of the one or more structures between the wire or band 20 and the expanded frame structure 12. The one or more structures may retain or nearly retain their normal anatomical position when the wire or band 20 is fully deployed.

Wire 20 may be formed of a material having sufficient rigidity to hold a predetermined shape. The wire may, for example, be formed of a shape memory material (e.g., NiTi). It may be desirable for at least an end portion (e.g., free end 22) to be relatively rigid such that it can exert a force to move chordal tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

Additional description for the anchor and other similar anchors usable in the embodiments described herein may be found in U.S. Patent Application No. 16/546,901, filed on August 21, 2019, entitled "PROSTHETIC CARDIAC VALVE DEVICES, SYSTEMS, AND METHODS," U.S. Patent Application No. 17/170,717, filed on February 8, 2021, entitled "PROSTHETIC CARDIAC VALVE DEVICES, SYSTEMS, AND METHODS," International Patent Application No. PCT/US2019/057082, filed on October 18, 2019, entitled "ADJUSTABLE MEDICAL DEVICE," U.S. Patent Application No. 16/723,537, filed on December 20, 2019, entitled "PROSTHETIC CARDIAC VALVE DEVICES, SYSTEMS, AND METHODS," U.S. Patent Application No. 16/824,576, filed on March 19, 2020, entitled "PROSTHETIC CARDIAC VALVE DEVICES, SYSTEMS, AND METHODS," International Patent Application No. PCT/US2020/027744, file on April 10, 2020, entitled "MINIMAL FRAME PROSTHETIC CARDIAC VALVE DELIVERY DEVICES, SYSTEMS, AND METHODS," and International Patent Application No. PCT/US2020/058413, filed on October 30, 2020, entitled "PROSTHETIC CARDIAC VALVE DEVICES, SYSTEMS, AND METHODS,".

One of ordinary skill in the art will recognize based on the description herein that any of the valve prostheses described herein may comprise any of the frame structure shapes, frame structure designs, frame structure materials, anchor shapes, anchor windings, anchor materials, free end tips, leaflet(s) configurations, or any other of the variable features described herein in any combination thereof as desired.

### Method of use

A valve prosthesis 10 may be loaded onto a delivery device 30, for example, a delivery catheter. The valve system may optionally be primed before or after loading onto the delivery device 30. The delivery device 30 may be inserted through an introducer into a vessel. The delivery device 30 can be guided over a guidewire to a target location, for example, using the Seldinger technique.

The distal end of the delivery device 30 may be configured to be advanced from a first side of a native valve to a second side of the native valve. For example, the distal end of the delivery device 30 may be advanced from a left atrial side of a mitral valve to a left ventricular side of a mitral valve. In some instances, the distal end of the delivery device 30 may be transseptally inserted into the left atrium of the heart prior to advancement into the left ventricle. Alternatively, or in combination, the distal end of the delivery device 30 may be steerable such that it is positionable to point towards the first side of the native valve before being advanced to the second side of the native valve.

Delivery device 30 may refer to various components in certain embodiments. For example, delivery device 30 may refer to a steerable catheter for transseptal delivery. Delivery device 30 may refer to a catheter for delivery of the replacement valve, frame structure, and/or anchor. In some embodiments, delivery device 30 may refer to a first delivery device component detachably coupled to the anchor and a second delivery device component detachably coupled to the frame structure. In some embodiments, one or more valve prosthesis components may be different delivery devices or different components of a delivery device system.

After advancing to the second side of the native valve, the anchor 15 may be fully deployed on the second side of the native valve. Fully deploying the anchor 15 may comprise actuating the anchor 15 from an elongated delivery configuration as shown in FIG. 1 to a deployed configuration as shown in FIG. 2.

In some embodiments, fully deploying the anchor 15 may comprise actuating the anchor 15 from an elongated delivery configuration to a deployed configuration on the first side of the native valve and advancing the anchor 15 in the deployed configuration through the native valve to the second side of the native valve. Advancing the anchor 15 may comprise pushing the anchor through the native valve. Advancing the anchor 15 may further comprise rotating the anchor 15m through the native valve.

In some embodiments, fully deploying the anchor 15 may comprise positioning the anchor 15 such that it is located only on the second side of the native valve.

In some embodiments, the anchor 15 may be actuated from the delivery configuration to the deployed configuration on a first side of the native valve prior to being advanced to a second side of the native valve. For example, the anchor 15 may be deployed in a left atrium of a heart prior to being advanced to a left ventricle of the heart as described herein.

Alternatively, the anchor 15 may be actuated from the delivery configuration to the deployed configuration on a second side of the native valve after being advanced to the second side from a first side of the native valve. For example, anchor 15 may be advanced from a left atrium of a heart prior to being deployed in a left ventricle of the heart.

The free end 22 of the deployed anchor 15 may optionally be rotated around one or more structures on the second side of the native valve. The one or more structures may comprise one or more valve leaflets of the native valve. Alternatively, or in combination, the one or more structures may comprise one or more chordae of the left ventricle.

The free end 22 of the deployed anchor 15 may optionally be rotated around one or more structures on the second side of the native valve such that the one or more structures (e.g., chordae, leaflets, or annulus) are pulled radially inwards towards the longitudinal axis of the anchor 15 and/or towards the longitudinal axis of the delivery device 30. The anchor 15 and/or free end 22 may be configured such that minimal torque is applied to the one or more structures. Alternatively, or in combination, the anchor 15 and/or free end 22 may be configured such that the one or more structures are not rotated, or are minimally rotated, during rotation of the anchor 15.

In some embodiments, the valve prosthesis 10, for example anchor 15, may be counter-rotated in order to reposition the anchor 15 with respect to the one or more structures of the native valve before continuing the rotation in the first direction. For example, counter-rotation may be applied if the one or more structures are caught by the free end of the anchor 15 (or another part of the valve prosthesis 10 or delivery device 30) during the initial rotation. In such instances, counter-rotation may enable to the clinician to disengage some or all of the one or more structures to reduce the stress or torque on the one or more structures (e.g., by adjusting the position of the valve prosthesis 10) before resuming rotation. Rotation and counter-rotation may be applied as many times as desired by the clinician in order to properly position the anchor 15 around the one or more structures of the native valve.

The anchor 15 may then be released from the distal end of the delivery device 30. The anchor 15 may be released from the distal end of the delivery device 30 on the second side of the native valve.

The frame structure 12 may be expanded within the native valve from an unexpanded configuration to an expanded configuration. The valve prosthesis 10 is finally anchored when the frame structure 12 is expanded within the native valve 4. The frame structure 12 dilates the valve leaflets 14 and the compressive force fixes the valve prosthesis 10 into position. Thereafter tissue ingrowth ensures the valve prosthesis 10 remains seated and does not migrate.

The frame structure 12 may be released from the distal end of the delivery device 30. In some embodiments, at least a portion the frame structure may be expanded within at least a portion of the deployed anchor to anchor the frame structure to the native valve.

In some embodiments, expanding the frame structure and releasing the frame structure may occur simultaneously.

Finally, the delivery device 30 may be retracted from the native valve.

The valve devices described herein in accordance with the present disclosure provides several advantages over conventional valve systems. Embodiments described herein provide an easy-to-use, repositionable device. Unlike conventional valve systems, the valve prosthesis described herein reduces the risk of injuring or tearing chordae. Typical mitral valve replacement systems involve implanting a prosthetic annulus or ring around the valve. The ring increases the circumference of the valve and risks occluding the entry to the aortic valve. The valve device described herein overcomes these and other problems.

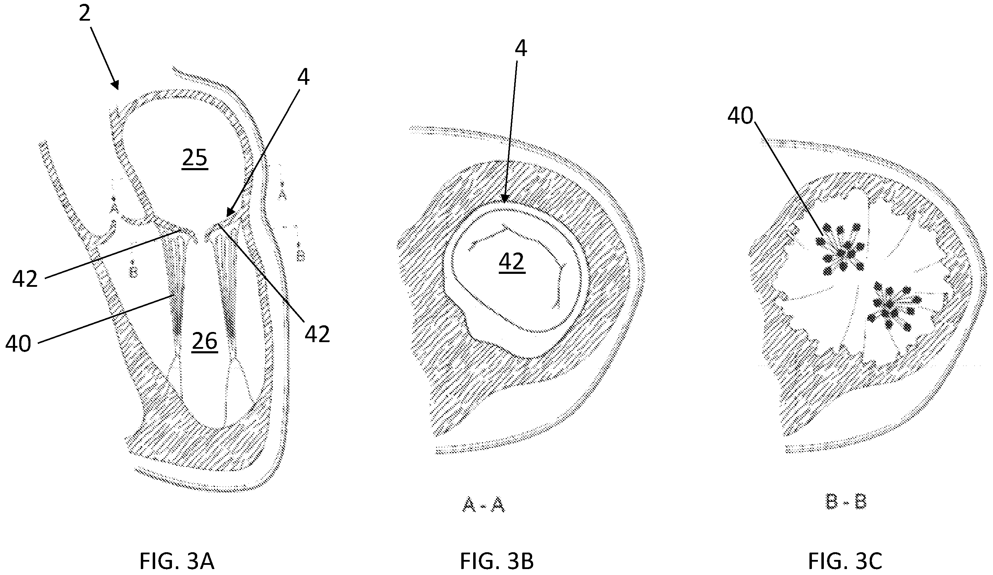

FIGS. 3A-3AE show sequential views of a method of implanting a valve prosthesis 10 using a delivery device 30. The valve prosthesis 10 may be similar to any of the valve prostheses described herein or understood by one of ordinary skill in the art from the description herein. For example, valve prosthesis 10 may be substantially similar to any of the valve prostheses 10 shown in at least FIGS. 1-2 and may comprise a frame structure 12 and anchor 15 as described herein. The delivery device 30 may be substantially similar to any of the delivery devices described herein or understood by one of ordinary skill in the art from the description herein. The delivery device 30 may comprise an inner shaft or delivery tube 52 as described herein. The delivery device 30 may optionally comprise an outer shaft or outer catheter 50, an inner shaft 52, and/or an inflatable balloon 48, in any combination thereof as desired by one of ordinary skill in the art. Not all elements are labeled in each of FIGS. 3A-3AE in order to make the illustrations less cluttered and easier to see.

While the method shown in FIGS. 3A-3AE is described in relation to a mitral valve replacement procedure, it will be understood by one of ordinary skill in the art that the methods described herein may be applied to a variety of procedures or anatomical areas, for example other atrioventricular valves of the heart or the like. For example, the methods described herein may be applied to replacement of a diseased aortic valve or tricuspid valve.

FIGS. 3A-3C shows various cross-sectional views of a heart 2 having a diseased mitral valve 4 which may be treated using the devices, systems, and methods described herein. The mitral valve 4 sits between the left atrium 25 and the left ventricle 26 and, when functioning properly, allows blood to flow from the left atrium 25 to the left ventricle 26 while preventing backflow or regurgitation in the reverse direction. As shown in FIG. 3A, the native valve leaflets 42 of the diseased mitral valve 4 do not fully prolapse and the patient experiences regurgitation. FIG. 3B shows a cross-sectional view of the heart 2 taken along line A-A, shown in FIG. 3A, which shows the native valve leaflets 42 of the mitral valve 4 from the viewpoint of the left atrium 25. FIG. 3C shows a cross-sectional view of the heart 2 taken along line B-B, shown in FIG. 3A, which shows the chordae tendineae 40 of the left ventricle 26.

As shown in FIG. 3D, a distal end of the delivery device 30 may be inserted into the left atrium 25 of the heart 2 via a transseptal puncture as described herein. For example, the distal ends of inner shaft 52 and/or outer sheath 50 may be advanced into the left atrium 25 of the heart 2. The inner shaft 52 may optionally be advanced distally into the left atrium 25 away from the distal end of the outer sheath 50. In some embodiments, advancing the inner shaft 52 relative to the outer sheath 50 may aid in deployment and/or placement of the valve prosthesis 10 as described herein. Alternatively, both the inner shaft 52 and the outer sheath 50 may be advanced distally into the left atrium 25 through the transseptal puncture.

FIGS. 3E-3H show deployment of the anchor 15 from the distal end of the delivery device 30. As described herein, at least a portion of the valve prosthesis 10 may be deployed from an undeployed (for example, compressed or unexpanded) configuration to an expanded configuration within the left atrium 25. At least a portion of the anchor 15 may be deployed from a delivery and/or elongated configuration to a deployed configuration within the heart. For example, anchor 15 may be actuated from an elongated configuration (e.g., from a straightened shaped) to a deployed configuration (e.g., a pre-formed shape for implantation, such as a spiral, helical, or conical shape) within the left atrium 25 as described herein. In some embodiments, the anchor 15 may be deployed from the inner shaft 52 by pushing the anchor 15 out of the inner shaft 52, releasing the anchor 15 from radial constraint by retracting the outer sheath 50, or the like as described herein. In some embodiments, the anchor 15 may be pushed out of the inner shaft 52 using a pusher on a proximal handle (not shown) located outside the body). After the anchor 15 has been deployed from the delivery device 30, the frame structure 12 may be at least partially deployed from the delivery device 30 as shown in FIG. 3H so as to place the frame structure 12 within the anchor 15. The frame structure 12 may be deployed from the delivery device 30 in either the unexpanded configuration or the expanded configuration, depending on the location of deployment, as will be understood by one of ordinary skill in the art based on the teachings herein.

FIGS. 3I-3K show advancement of the valve prosthesis 10, with anchor 15 deployed around the unexpanded frame structure 12, towards the native valve 4 requiring treatment. The distal end of the delivery device 30 (for example, the distal end of the inner shaft 52 and/or the outer sheath 50) may be steered such that the distal end of the delivery device 30 points toward the atrial side of the native valve 4. Such steering may occur prior to, during, or after deployment of at least a portion (for example deployment of an anchor 15) of the valve prosthesis 10. In some embodiments, the distal end of the outer sheath 50 may be steerable. Alternatively, or in combination, the inner shaft 52 may comprise a joint configured to change an angle of the distal portion of the inner shaft 52 relative to a proximal portion of the inner shaft 52. The inner shaft 52 may be steered by changing the angle of the distal portion of the inner shaft 52 relative to the proximal portion of the inner shaft 52. The angle of the joint may be changed passively or actively. In various embodiments, the angle may be selectively controlled by a proximal handle. For example, pull wires or other mechanisms may connect to the joint to controls on the handle.

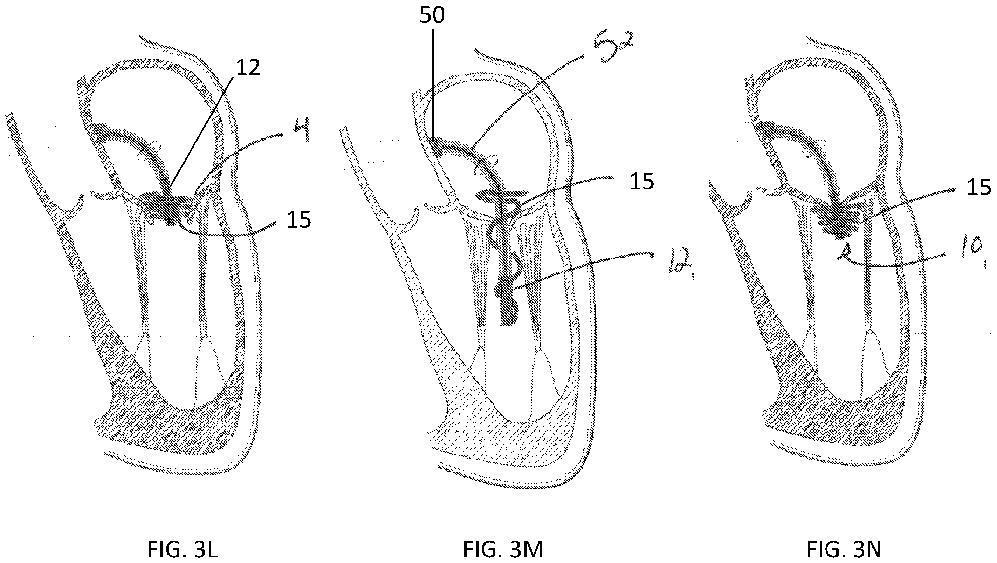

FIGS. 3L-3O show the valve prosthesis 10 being advanced through the native valve 4 by the delivery device 30 from the left atrium 25 to the left ventricle 26. Advancement of the valve prosthesis 10 and optionally delivery device 30 through the mitral valve 4 may be facilitated by the natural opening and closing of the valve 4 during the cardiac cycle. The distal end of the delivery device 30 and/or valve prosthesis 10 may be configured to be advanced from a first side of a native valve to a second side of the native valve. For example, the distal end of the delivery device 30 and/or valve prosthesis 10 may be advanced from a left atrial side of a mitral valve 4 to a left ventricular side of a mitral valve 4. Advancing the anchor 15 may comprise pushing the anchor 15 through the native valve 4. Alternatively, or in combination, advancing the anchor 15 may comprise rotating the anchor 15 through the native valve 4. In some instance, the combination of rotational motion and pushing may facilitate advancement of the device from the first side of the native valve 4 to the second side of the native valve 4. Rotation of the valve prosthesis 10, for example rotation of the anchor 15 and/or frame structure 12, may be facilitated by the inner shaft 52 described herein. For example, the inner shaft 52 may transmit rotational motion to the valve prosthesis 10 in order to rotate the valve prosthesis 10 during advancement through the native valve 4.

In some instances, advancing the anchor 15 through the native valve 4 may cause the anchor 15 to be stretched or elongated as shown in FIG. 3M. Rotation of the anchor 15 during advancement may assist with the stretching process by aiding in unwinding the anchor 15. Additionally, the rotational motion may reduce the risk of the free end 22 of the anchor 15 undesirably engaging other anatomy during insertion through the native leaflets 43. The anchor 15 may be sufficiently elastic so as to enable relatively easy insertion through the native valve 4 and/or reduce the risk of injury to the native leaflets 43. At the same time, the anchor may be sufficiently rigid to for guiding through and anchoring to the structures in the heart. After the anchor 15 has stretched through the native valve 4 it may return to the deployed configuration as shown in FIG. 3N. FIG. 3O shows the position of the valve prosthesis 10 within the ventricle 26 and, in particular, the position of the anchor 15 relative to the native chordae tendineae 40 and native valve annulus.

In some embodiments, the anchor 15 may be advanced into the ventricle after being fully deployed from the delivery (e.g., elongated) configuration to the deployed configuration.

In some embodiments, the anchor 15 may be advanced into the ventricle before being deployed from the delivery (e.g., elongated) configuration to the deployed configuration.

FIGS. 3P-3S show rotation of the valve prosthesis 10 around one or more structures on the ventricular side of the mitral valve 4. The one or more structures may comprise one or more valve leaflets 43 and/or one or more chordae tendineae 40. After the anchor 15 has been at least partially deployed within the left ventricle 26 adjacent one or more chordae tendineae 40, the valve prosthesis 10 may be rotated to capture and anchor the native chordae 40 and/or native leaflets 43. The free end 22 of the anchor 15 may extend radially outward from the rest of the anchor 15 to facilitate capture of the native structures. The free end 22 of the anchor 15 may be rotated around one or more of the chordae tendineae 40 as shown in FIGS. 3P-3Q. Additional rotation of the valve anchor 15 may gradually capture additional chordae tendineae 40 as shown in FIGS. 3R-3S.

Rotation of the valve prosthesis 10, for example, rotation of the anchor 15 and/or frame structure 12, may be facilitated by the delivery device 30 described herein. For example, the inner shaft 52 may be rotated and rotational motion may be transmitted from the inner shaft 52 to the valve prosthesis 10 in order to rotate the valve prosthesis 10 around one or more of the structures on the ventricle side of the mitral valve 4 as described herein.

In some embodiments, all or a portion of the valve prosthesis 10, for example anchor 15, may be counter-rotated in order to reposition the anchor 15 with respect to the chordae tendineae 40 before continuing the rotation in the first direction. For example, counter-rotation may be applied if the chordae tendineae 40 are caught by the free end of the anchor 15 (or another part of the valve prosthesis 10 or delivery device 30) during the initial rotation. In such instances, counter-rotation may enable to the clinician to disengage some or all of the chordae tendineae 40 to reduce the stress or torque on the chordae tendineae 40 (e.g., by adjusting the position of the valve prosthesis 10) before resuming rotation. As another example, the anchor 15 may encounter friction or other resistance to rotation. In this case the clinician may counter-rotate the anchor 15 to return to the original position and then begin rotating the anchor 15 to re-start and/or continue encircling chordae tendineae 40. Rotation and counter-rotation may be applied as many times as desired by the clinician in order to properly position the anchor 15 around the valve structures.

FIGS. 3T-3U show the valve prosthesis 10 wrapped around the captured chordae tendineae 40. The valve prosthesis 10 may be rotated around the chordae tendineae 40 such that the chordae tendineae 40 are pulled inwardly into bunches. As shown in FIG. 3T, the native valve leaflets 43 may also be in communication with the valve prosthesis 10. The valve prosthesis 10 may be rotated to capture enough chordae tendineae 40 and/or valve leaflets 43 to rigidly anchor the anchor 15 adjacent the native valve annulus. The valve prosthesis 10 may be anchored by wrapping around only a portion of the chordae 40. Although it may be possible to capture all or substantially all the chordae 40, this may not be necessary to provide sufficient anchoring of the valve prosthesis 10. As described further herein, the prosthesis may be further anchored by expansion of the frame structure 12 within the native valve 4 and against the anchor 15. Alternatively, or in combination, the anchor may be sufficiently anchored after winding around some or only a portion of the chordae. It is believed that only a portion of the chordae are necessary for anchoring.

In some embodiments, the anchor 15 may be deployed such that at least a portion of the anchor 15 resides within a subvalvular plane. For example, at least 50 %, 60%, 70%, 80%, 90%, 100% of the anchor 15 may reside within the subvalvular plane after being deployed. The subvalvular plane may be located at the posterior valve annulus, below the valve annulus and around the native valve leaflets 43, and/or parallel to a plane within at least three points of the plane in which the valve annulus resides. In some instances, the anchor 15may be rotated in the subvalvular plane around the chordae tendineae 40. In some instances, the anchor 15 may be rotated in a plane below to the subvalvular plane in order to encircle the chordae tendineae 40 before the anchor 15 is moved into the subvalvular plane (e.g., by pulling the anchor 15 into the sub-annular space).

Once the anchor 15 has been anchored adjacent to the native valve 4, the frame structure 12 and prosthetic valve segment 14 may be expanded at least partially within the anchor 15 as described herein. The frame structure 12 and the valve segment 14 may be deployed (e.g., expanded) simultaneously. Alternatively, or in combination, the frame structure 12 and the valve segment 14 may be deployed sequentially, for example by first expanding the frame structure 12 and then receiving the prosthetic valve segment 14 therein.

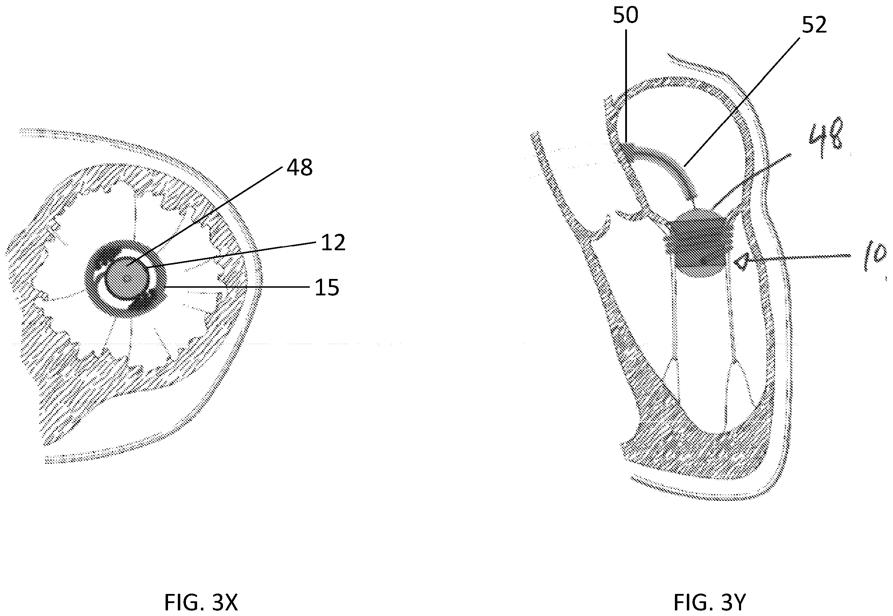

FIGS. 3V-3Z show expansion of the frame structure 12 within the native valve 4. The frame structure 12 may be expanded within the native valve 4 from an unexpanded configuration to an expanded configuration. In some embodiments, at least a portion the frame structure 12 may be expanded within at least a portion of the deployed anchor 15 to anchor the frame structure 12 to the native valve 4. In some embodiments, the frame structure 12 may comprise an expandable stent. In some embodiments, the frame structure 12 of valve prosthesis 10 may be self-expandable. In some embodiments, the frame structure 12 of valve prosthesis 10 may be balloon-expandable. The delivery device 30 may comprise a balloon 48 which may be disposed within the valve prosthesis 10 in order to expand the valve prosthesis 10. The balloon 48 may be positioned within at least a portion of the valve prosthesis 10, for example within at least a portion of frame structure 12 in an uninflated configuration, as shown in FIG. 3V, prior to being inflated. The inflatable balloon 48 may, for example, be disposed within the inner shaft 52 or outer sheath 50 while the anchor 15 is being positioned adjacent the native valve 4 and then advanced therefrom (or the inner shaft 52 or outer sheath 50 is retracted therefrom) to be positioned within the frame structure 12. Alternatively, the inflatable balloon 48 may be disposed within the frame structure 12 during placement of the valve prosthesis 10. FIGS. 3W-3X show the frame structure 12 partially expanded by partially-inflated balloon 48. As shown in FIG. 3X, the frame structure 12 may be partially expanded towards the anchor 15 in order to capture the chordae tendineae 40 therebetween. As the frame structure 12 continues to be expanded to a fully expanded state, as shown in FIGS. 3Y-3Z, the chordae tendineae 40 may be sandwiched between the anchor 15 and the frame structure 12. The frame structure 12 and anchor 15 may thus be anchored to the chordae tendineae 40.

The valve prosthesis 10 may then be released from the delivery device 30. In some embodiments, releasing the valve prosthesis 10 may comprise releasing the anchor 15 and/or the frame structure 12. Releasing the valve prosthesis 10 from the delivery device 30 may comprise expanding the valve prosthesis 10 from the unexpanded configuration to the expanded configuration. For example, expanding the frame structure 12 and releasing the frame structure 12 may occur simultaneously as described herein. Alternatively, the frame structure 12 may be released prior to or after being expanded.

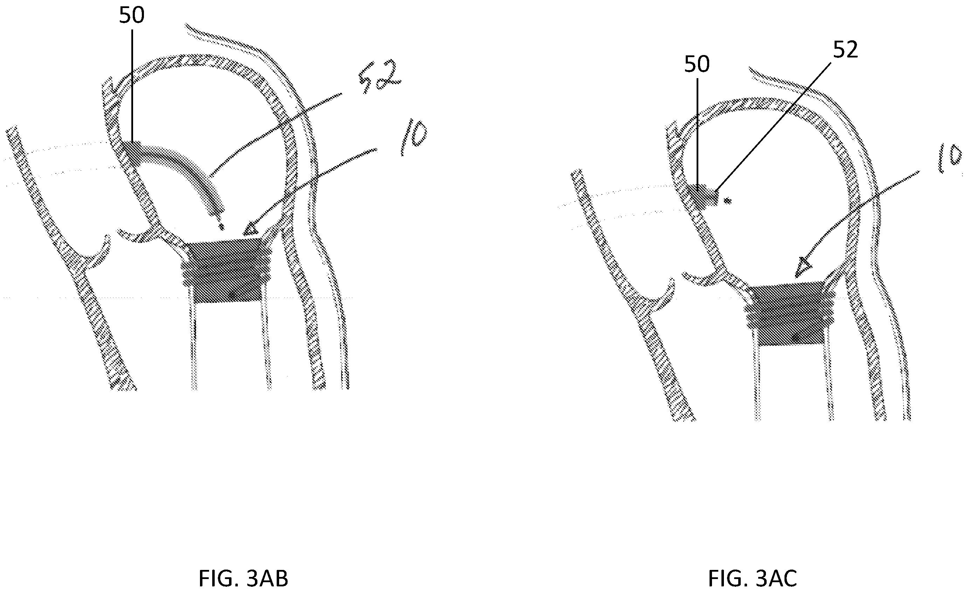

FIGS. 3AA-3AC show deflation of the balloon 48 (FIG. 3AA), retraction of the balloon 48 into inner shaft 52 (FIG. 3AB), and removal of the delivery device 30 from the heart 2 (FIG. 3AC). After the frame structure 12 has been expanded and anchored to the native valve 4 as described herein, the inflatable balloon 48 may be deflated. The balloon 48 may optionally be retracted back into the delivery device 30, for example into inner shaft 52. The delivery device 30 may then be removed from the heart 2.

FIGS. 3AD-3AE show the valve prosthesis 10 fully expanded with the native valve leaflets 42 and chordae tendineae 40 captured between the frame structure 12 and the anchor 15. As described herein, the valve prosthesis 10 may comprise one or more valve segments 14 disposed therein to replace the native valve leaflets 42.

Although the steps above show a method of deploying a valve prosthesis 10 within a native valve 4 in accordance with embodiments, a person of ordinary skill in the art will recognize many variations based on the teaching described herein. The steps may be completed in a different order. Steps may be added or deleted. Some of the steps may comprise sub-steps. Many of the steps may be repeated as often as necessary to assemble at least a part of an article.

For example, in some embodiments deploying the valve prosthesis 10 may occur in multiple steps such that a portion of the valve prosthesis 10 (e.g., anchor 15) may be deployed before another portion the valve prosthesis 10 (e.g., frame structure 12). Alternatively, or in combination, in some embodiments, deploying the anchor 15 may occur in multiple steps such that a portion of the anchor 15 may be deployed before being advanced through the native valve 4 and another portion of the anchor 15 may be deployed after being advanced through the native valve 4. Alternatively, or in combination, the delivery device 30 may be advanced from the left atrium 25 to the left ventricle 26 with the valve prosthesis 10 undeployed. In many embodiments, the frame structure may 12 be self-expanding and the balloon 48 may not be necessary for expansion of the frame structure 12. Alternatively, or in combination, the anchor 15 may be released after the frame structure 12 has been expanded within it.

Although shown and described with respect to a mitral valve, one of ordinary skill in the art will understand that the principles described herein may be applied equally to other atrioventricular valves.

In some embodiments, any of the valve prostheses described herein may be deployed to replace a diseased mitral valve. The first side of the native valve may comprise a left atrium and the second side of the native valve may comprise a left ventricle.

In some embodiments, any of the valve prostheses described herein may be deployed to replace a diseased tricuspid valve. The first side of the native valve may comprise a right atrium and the second side of the native valve may comprise a right ventricle.

In some embodiments, any of the valve prostheses described herein may be deployed to replace a diseased aortic valve. The first side of the native valve may comprise a left ventricle and the second side of the native valve may comprise an aorta.

The valve prosthesis device and implant method described herein in accordance with the present disclosure may provide many advantages as will be understood by one of ordinary skill in the art. The overall device and method may provide a simpler way to approach the native valve compared to existing devices. The system may enable a transcatheter approach through the septal wall compared to more invasive transapical approaches. The device may provide a consistent and relatively easy mechanism for anchoring to the native valve. Clinicians need only use the common technique of inserting the device through the valve and then rotating the anchor. The coil may provide preliminary anchoring in the native valve. If desired, the clinician can readjust the anchor and/or retrieve the anchor (e.g. by counterrotation). The device is then easily set by expanding within the native valve leaflets. The device and methods in accordance with the present disclosure may also address unmet clinical needs with atrioventricular repair and replacement. Existing devices face challenges with the complex anatomy of the mitral and tricuspid valves, for example. The present disclosures address these complications by reshaping the native valve annulus to a conventional round shape and providing a robust, yet simple, anchoring mechanism.

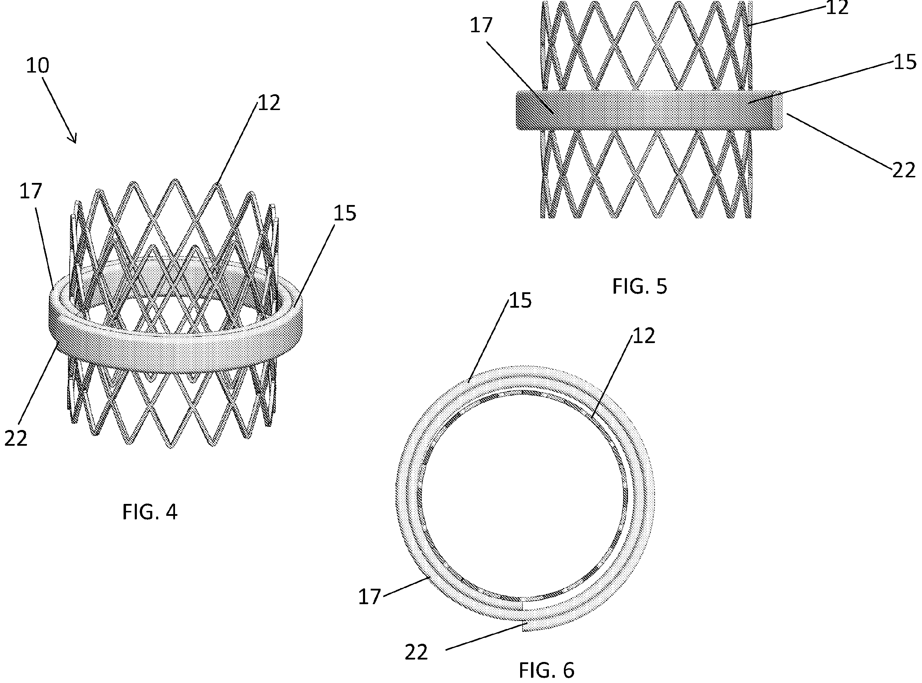

FIGS. 4-6 show several views of a valve prosthesis 10 with an anchor 15 comprising a flat spiral shaped anchor. The valve prosthesis 10 may be substantially similar to any of the valve prostheses described herein except that the anchor may include a flat spiral band 17. In some embodiments, the band 17 can have a larger height in the axial direction than width in the radial direction when deployed. The frame structure 12 may have an unexpanded configuration (for example, a compressed configuration as described herein) and an expanded configuration as described herein. The frame structure 12 may be substantially similar to any of the frame structures described herein or understood by one of ordinary skill in the art from the description herein. The anchor 15 may be configured to anchor the frame structure 12 to the native valve when the frame structure 12 is in the expanded configuration adjacent the native valve. The frame structure 12 may be configured to be actuated from the unexpanded configuration to the expanded configuration adjacent a native valve in a patient. The anchor 15 may be configured to be fully advanced from a first side of a native valve in a patient (e.g. an atrial side) to a second side of the native valve (e.g. into a ventricle of the heart) and anchor the frame structure 12 to the native valve when the frame structure 12 is in the expanded configuration adjacent the native valve as described herein.

The flat spiral anchor 15 may comprise a free (distal) end 22. The other end of the anchor 15 may be coupled to the top (proximal end) or bottom (distal end) of the frame structure 12 as described herein. Alternatively, or in combination, the other end of the anchor 15 may not be attached to the frame structure 12 as described herein. The free end 22 of the anchor 15 may facilitate capturing of the valve leaflets and/or chordae tendineae within the sweep of the free end during rotation as described herein. During rotation of the anchor 15, the leaflets and/or chordae tendineae may be captured by the free end 22 and trapped between the valve frame structure 12 and an interior surface of the anchor 15.

The free end 22 of the flat spiral anchor 15 may be sized and dimensioned for insertion through the native valve, for example through tissue at or near a commissure of the native valve or through the valve opening itself. In some embodiments, the free end 22 may comprise an atraumatic tip to avoid reduce risk of injury to the native valve tissue and leaflets. For example, the free end 22 may comprise a blunt end, a ball tip, a curved tip (e.g. J-tip or pigtail), or other atraumatic shapes. Alternatively, the free end 22 may be configured for piercing tissue. In various embodiments, the free end 22 is separate from and extends outward from the main coils of the anchor 15. In various embodiments, the main body coils of the anchor 15 circumscribe an area (in the case of a spiral coil) or a volume (in the case of a helical coil) having a diameter, and the free end 22 extends to a radius greater than the diameter of the anchor 15. In various embodiments, the free end 22 extends to a radius substantially greater than the diameter of the anchor 15. In various embodiments, the free end 22 is configured to circumscribe a larger diameter than the anchor 15. In various embodiments, the free end 22 is configured to circumscribe all of the chordae tendineae of the native valve to be treated.

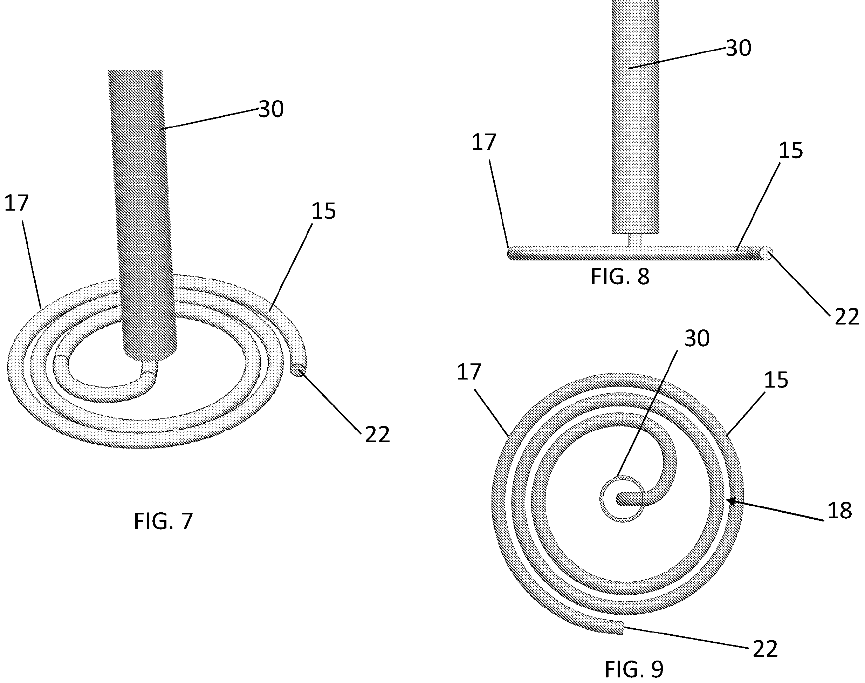

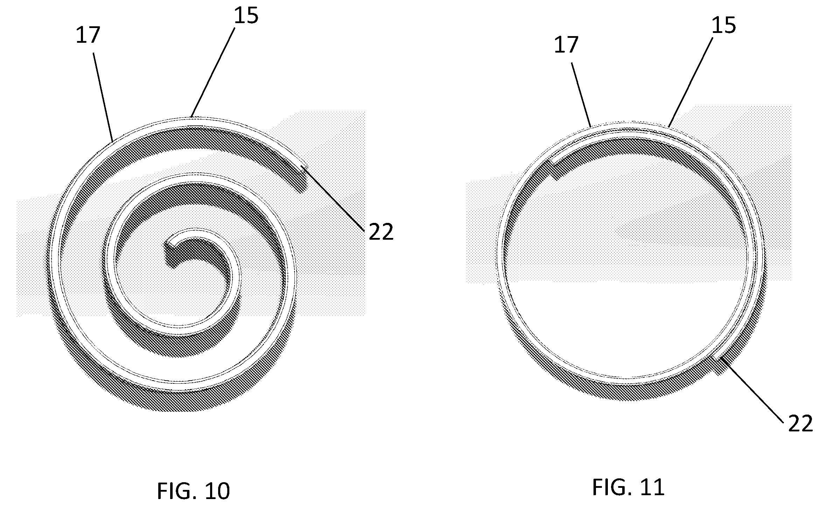

The free end 22 of the flat spiral anchor 15 may optionally rotated around one or more structures on the second side of the native valve such that the one or more structures (e.g., chordae, leaflets, or annulus) are pulled radially inwards towards the longitudinal axis of the anchor 15 and/or towards the longitudinal axis of the delivery device 30. The anchor 15 and/or free end 22 may be configured such that minimal torque is applied to the one or more structures. Alternatively, or in combination, the anchor 15 and/or free end 22 may be configured such that the one or more structures are not rotated, or are minimally rotated, during rotation of the anchor 15. For example, the anchor 15 may comprise one or more spaces between loops of the spiral band (for example, spaces 18 shown in FIG. 9) which facilitate movement of the captured tissue (e.g. chordae and/or leaflets) from the free end 22 to the center of the spiral structure with little or no torque and/or rotation of the structures during rotation of the anchor 15 as described herein. Alternatively, or in combination, the anchor 15 may be configured such that, when fully deployed (for example, as shown in FIG. 11), none of the structures reside between the loops of the spiral. Instead, the one or more structures may sit radially inward of the loops in order to facilitate capture of the one or more structures between the anchor 15 and the expanded frame structure 12. The one or more structures may retain or nearly retain their normal anatomical position when the anchor 15 is fully deployed.

The anchor 15 may comprise a delivery (e.g. an elongated) configuration and a deployed configuration. The anchor 15 may be configured to be actuated from the delivery configuration to the deployed configuration adjacent a native valve in a patient. In various embodiments, the anchor 15 may have a generally spiral shape in the deployed configuration. In various embodiments, the anchor 15 may be elongated-rather than spiral-shaped-in the delivery configuration. For example, the anchor 15 may be elongated into a straight shape within the delivery device. In various embodiments, a portion of the anchor 15 may have a spiral shape. In various embodiments, a substantial portion of the anchor 15 may have a spiral shape. In various embodiments, the spiral anchor 15 may be formed as a flat spiral (in the deployed configuration) whereby the loops generally are positioned within the same plane (the plane being perpendicular to a longitudinal axis).

Anchor 15 may be formed of a material having sufficient rigidity to hold a predetermined shape. The wire may, for example, be formed of a shape memory material (e.g. NiTi). It may be desirable for at least an end portion (e.g. free end 22) to be relatively rigid such that it can exert a force to move chordae tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

The anchor 15 may comprise a planar spiral band 17. The anchor 15 may comprise a spiral band 17 having a free end 22. The other end of the spiral band 17 may be coupled to the top (proximal end) or bottom (distal end) of the frame structure 12 as described herein. Alternatively, or in combination, the other end of the spiral band 17 may not be attached to the frame structure 12 as described herein. The free end 22 of the spiral band 17 may facilitate capturing of the valve leaflets and/or chordae tendineae within the sweep of the free end during rotation as described herein. During rotation of the spiral band 17, the leaflets and/or chordae tendineae may be captured by the free end 22 and trapped between the valve frame structure 12 and an interior surface of the spiral band 17.

The free end 22 of the spiral band 17 may be sized and dimensioned for insertion through the native valve, for example through tissue at or near a commissure of the native valve or through the valve opening itself. In some embodiments, the free end 22 may comprise an atraumatic tip to avoid reduce risk of injury to the native valve tissue and leaflets. For example, the free end 22 may comprise a blunt end, a ball tip, a curved tip (e.g. J-tip or pigtail), or other atraumatic shapes. Alternatively, the free end 22 may be configured for piercing tissue. In various embodiments, the free end 22 is separate from and extends outward from the main coils of the anchor 15. In various embodiments, the main body coils of the anchor 15 circumscribe an area (in the case of a spiral coil) or a volume (in the case of a helical coil) having a diameter, and the free end 22 extends to a radius greater than the diameter of the anchor 15. In various embodiments, the free end 22 extends to a radius substantially greater than the diameter of the anchor 15. In various embodiments, the free end 22 is configured to circumscribe a larger diameter than the anchor 15. In various embodiments, the free end 22 is configured to circumscribe all of the chordae tendineae of the native valve to be treated.

The free end 22 of the spiral band 17 may optionally rotated around one or more structures on the second side of the native valve such that the one or more structures (e.g., chordae, leaflets, or annulus) are pulled radially inwards towards the longitudinal axis of the anchor 15 and/or towards the longitudinal axis of the delivery device 30. The spiral band 17 and/or free end 22 may be configured such that minimal torque is applied to the one or more structures. Alternatively, or in combination, the spiral band 17 and/or free end 22 may be configured such that the one or more structures are not rotated, or are minimally rotated, during rotation of the spiral band 17. For example, the spiral band 17 may comprise one or more spaces between loops of the spiral band (for example, spaces 18 shown in FIG. 9) which facilitate movement of the captured tissue (e.g. chordae and/or leaflets) from the free end 22 to the center of the spiral structure with little or no torque and/or rotation of the structures during rotation of the spiral band 17 as described herein. Alternatively, or in combination, the spiral band 17 may be configured such that, when fully deployed (for example, as shown in FIG. 11), none of the structures reside between the loops of the spiral. Instead, the one or more structures may sit radially inward of the loops in order to facilitate capture of the one or more structures between the spiral band 17 and the expanded frame structure 12. The one or more structures may retain or nearly retain their normal anatomical position when the spiral band 17 is fully deployed.

The spiral band 17 may comprise a delivery (e.g. an elongated) configuration and a deployed configuration. The spiral band 17 may be configured to be actuated from the delivery configuration to the deployed configuration adjacent a native valve in a patient. In various embodiments, the band 17 may have a generally spiral shape in the deployed configuration. In various embodiments, the band 17 may be elongated-rather than spiral-shaped-in the delivery configuration. For example, the band 17 may be elongated into a straight shape within the delivery device. In various embodiments, a portion of the band 17 may have a spiral shape. In various embodiments, a substantial portion of the band 17 may have a spiral shape. In various embodiments, the spiral band 17 may be formed as a flat spiral (in the deployed configuration) whereby the loops generally are positioned within the same plane (the plane being perpendicular to a longitudinal axis).

Spiral band 17 may be formed of a material having sufficient rigidity to hold a predetermined shape. The wire may, for example, be formed of a shape memory material (e.g. NiTi). It may be desirable for at least an end portion (e.g. free end 22) to be relatively rigid such that it can exert a force to move chordae tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

FIGS. 7-9 show several views of the flat spiral anchor 15 of FIGS. 4-6 loaded on a delivery device 30. The delivery device 30 may be substantially similar to any of the delivery devices described herein. For example, the delivery device 30 may comprise an outer sheath in which the anchor 15 may be disposed, for example in an elongated or undeployed delivery configuration as described herein. Alternatively, or in combination, the delivery device 30 may comprise an inner shaft, for example disposed within the lumen of the outer sheath. The anchor 15 and/or frame structure 12 may be coupled to the inner shaft as described herein. The delivery device 30 may comprise a guidewire as described herein. The delivery device 30 may be configured to deliver the anchor 15 to the native valve as described herein. The manner of implanting valve device 10 may be substantially similar to any of the methods described herein.

The flat spiral anchor 15 (e.g., spiral band 17) may comprise one or more loops. For example, the anchor 15 may comprise a plurality of loops, which may increase the radial strength of the anchor by increasing friction and addition structural support. The one or more loops of the anchor 15 may be spiral radially outward from a central point or central axis of the spiral, for example along an axis which is coaxial with a longitudinal axis of a delivery device 30 such that the spiral anchor 15 lies approximately along a plane perpendicular to the longitudinal axis of the delivery device 30.

The one or more loops (also referred to herein as coils or turns) of the flat spiral anchor 15 (e.g., spiral band 17) may comprise a shape that bends around back towards its origin (for example, an arc, ellipsoid, circle, or the like). In some embodiments, a loop may comprise a shape that bends back towards its origin but does not cross itself, making a rotation within a range of about 180 degrees to about 360 degrees. For example, a loop may comprise an arc having a central angle within a range of about 180 degrees to about 360 degrees. In at least some embodiments, the one or more loops may comprise an arc. In some embodiments, a loop may comprise a shape that bends back towards and crosses itself, making at least a 360 degree rotation. In at least some embodiments, the one or more loops may comprise a 360 degree rotation (for example, a circle). In some embodiments, the one or more loops may comprise a 360 degree to 720 degree rotation (for example, a loop crossing itself once and rotating further towards a second crossing and formation of a second 360 loop).

The one or more loops may comprise any number of loops desired, for example, one, two, three, four, five, six, seven, eight, nine, or ten loops. The one or more loops may comprise a rotation within a range of about 180 degrees to about 3600 degrees. The one or more loops may comprise a rotation within a range bounded by any of the following values: 180 degrees, 270 degrees, 360 degrees, 450 degrees, 540 degrees, 630 degrees, 720 degrees, 810 degrees, 900 degrees, 990 degrees, 1080 degrees, 1170 degrees, 1260 degrees, 1350 degrees, 1440 degrees, 1530 degrees, 1620 degrees, 1710 degrees, 1800 degrees, 1890 degrees, 1980 degrees, 2070 degrees, 2160 degrees, 2250 degrees, 2340 degrees, 2430 degrees, 2520 degrees, 2610 degrees, 2700 degrees, 2790 degrees, 2880 degrees, 2970 degrees, 3060 degrees, 3150 degrees, 3240 degrees, 3330 degrees, 3420 degrees, 3510 degrees, or 3600 degrees.

In some embodiments, for example when the free end 22 is disposed at an angle (upward, downward, or radially outward) relative to the rest of the anchor body, the main body of the anchor comprises the one or more loops and the rotation of the one or more loops may be determined without taking into account the angled free end 22.

Interaction of the frame structure 12 with the one or more loops of the anchor 15 may create opposing forces therebetween that provide mechanical leverage for anchoring the frame structure to the one or more anatomical structures. In some embodiments, the one or more loops may comprise at least 360 degrees of rotation (e.g., 540 degrees) when deployed such that the loops wrap around one another and provide additional mechanical leverage against the frame structure in order to facilitate anchoring of the frame structure as described herein. Additional loops or partial loops may provide additional mechanical strength and/or leverage.

In some embodiments, the one or more loops may comprise a rotation of about 540 degrees. A first loop may comprise a rotation of 360 degrees and a second loop may comprise a rotation of about 180 degrees. The second loop may wrap around the first loop for about 180 degrees in order to provide additional mechanical strength and/or leverage against the frame structure in order to facilitate anchor of the frame structure. In some instances, the one or more loops comprising a rotation of about 540 degrees may facilitate easier and safer capture of the chordae tendineae and/or valve leaflets than an anchor having additional loops by providing sufficient mechanical strength and/or leverage and/or radial stability while reducing or minimizing the amount of overlap between loops in which to potentially ensnare or entangle the native valve structures during rotation.

In some embodiments, the one or more loops of the anchor 15 may comprise one or more spaces 18 therebetween. The spaces 18 may facilitate movement of the captured tissue (e.g. chordae and/or leaflets) from the free end 22 to the center of the spiral structure during rotation of the anchor 15 as described herein.

The anchor 15 (e.g., spiral band 17) may comprise a spiral wire. The anchor 15 may comprise a plurality of spiral wires as described herein.

In some embodiments, the delivery device 30 may be configured to carry the anchor 15 in an undeployed configuration and deploy the anchor 15 into a deployed configuration as the desired location as described herein.

In some embodiments, the anchor 15 may be configured to wrap at least partially around a distal portion of the delivery device 30, for example around the inner shaft, in the deployed configuration.

In some embodiments, the anchor 15 may comprise one or more locking mechanisms configured to maintain the anchor 15 in the deployed configuration. The one or more locking mechanisms may be any of the locking mechanisms described herein or understood by one of ordinary skill in the art from the description herein. In various embodiments, one or more loops may be nested with each other when the anchor is in the deployed configuration.

FIGS. 10-11 show deployment of the anchor of FIGS. 4-6 from an undeployed configuration (FIG. 10) to a deployed configuration (FIG. 11). The anchor 15 may comprise an undeployed configuration (shown in FIG. 10) and a deployed configuration (shown in FIG. 11). The anchor 15 may be configured to be actuated from the undeployed configuration to the deployed configuration adjacent the native valve. The anchor 15 may comprise a spiral band 17. The anchor 15 may comprise a flat spiral or planar spiral shape in the deployed configuration. The free end 22 of the anchor 15 may extend radially outward from the frame 12, and in particular from the remainder of the anchor 15. In some embodiments, expansion of the frame structure 12 inside the anchor 15 may cause the anchor 15 to expand from the undeployed configuration to the deployed configuration. Alternatively, or in combination, rotation of the anchor 15 around one or more structures as described herein may cause the anchor 15 to expand from the undeployed configuration to the deployed configuration.

In some embodiments, the anchor 15 may comprise a delivery (e.g., elongated) configuration when disposed within a delivery device as described herein. The delivery device may be configured to deploy the anchor 15 from the elongated configuration to a first intermediate deployed configuration (substantially similar to the undeployed configuration shown in FIG. 10) as described herein. The anchor 15 may then be actuated into the second fully deployed configuration (substantially similar to the deployed configuration shown in FIG. 11), for example by expanding the frame structure within the anchor 15 or by rotating the anchor 15 to capture the one or more structures as described herein.

The anchor 15 may comprise one or more loops of a spiral as described herein. The anchor 15 may comprise a more compact spiral, with more loops, in the undeployed configuration compared to the deployed configuration. As the anchor 15 expands the spiral loops may unwind.

In some embodiments, the outer perimeter of the anchor 15 (e.g., spiral band 17) may be substantially similar in the undeployed and deployed configurations. As the spiral loops unwind, the spiral may expand from the center, for example simultaneously with expansion of the frame structure in the center of spiral anchor 15, such that the spiral loops are expanded against one another as shown in FIG. 11. As will be understood by one of ordinary skill in the art, the shape, size, and number of loops may determine the limits of expansion of the spiral anchor 15 and alteration of any or all of these parameters may be done to generate a final deployed configuration desired from an initial undeployed configuration.

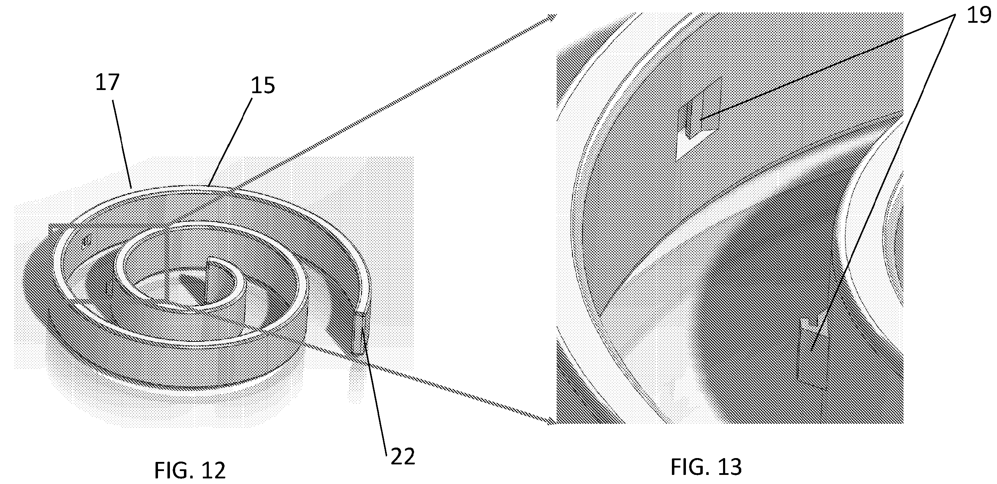

FIGS. 12-13 show an optional locking mechanism for a spiral band anchor. Anchor 15 may comprise a spiral band 17. The anchor 15 may comprise one or more optional locking features disposed along the spiral loops such that they interlock when expanded against one another. For example, the locking features may comprise one or more "key-in-hole" (e.g., key and key hole) interlocking features 19 as shown which lock the spiral anchor 15 in a deployed configuration when the spiral loops are expanded such that the two corresponding features meet and connect. In some instances, it may be beneficial to provide the spiral anchor 15 with a plurality of locking features, for example to ensure secure locking and/or to provide for a plurality of intermediate expanded/deployed configurations between the fully undeployed and the fully deployed configuration. It will be understood by one of ordinary skill in that any number or style of locking features may be used to lock the spiral anchor 15 into a deployed (and/or intermediately deployed) configuration.

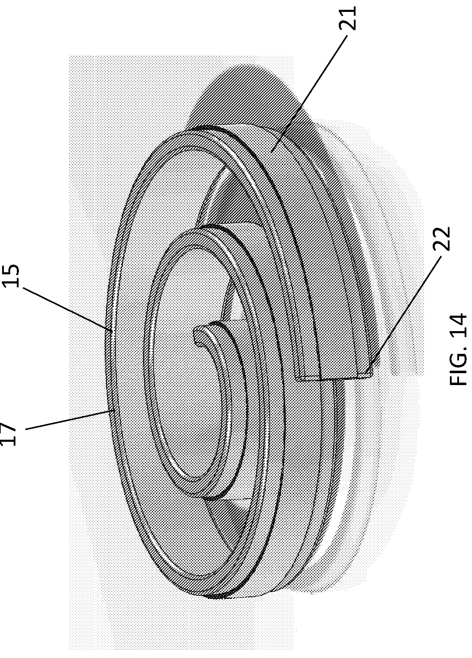

FIG. 14 shows another optional locking mechanism for a spiral band anchor. Anchor 15 may comprise a spiral band 17. The anchor 15 may comprise an optional frictional band 21 disposed upon a length of the anchor 15 which may facilitate locking the anchor 15 in a deployed configuration as the spiral loops expand. By adding a frictional texture or band 21 to at least a portion of the anchor 15, the loops may be encouraged to engage one another as they are expanded against each other.

The frictional band 21 may, for example comprise a strip of frictional material that may be bonded to the surface of the anchor 15 by any method understood by one of ordinary skill in the art from the description herein.

Alternatively, or in combination, at least a portion of the anchor 15 may be coated with a polymer and the outer surface of the polymer may be made rough using any technique understood by one of ordinary skill in the art from the description herein.

Alternatively, or in combination, the surface of the anchor 15 may be directly altered for increased friction to form the frictional band 21 using any technique understood by one of ordinary skill in the art from the description herein.

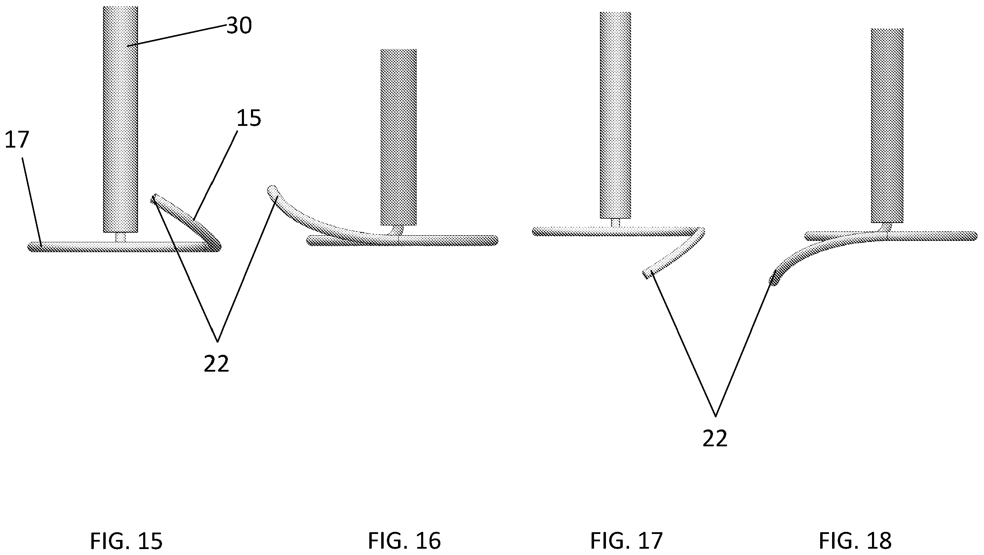

FIGS. 15-16 show an optional tip orientation for a spiral anchor. In some embodiments, the spiral anchor 15, which may comprise a spiral band 17, may comprise a free end 22 which is angled or bent such that it points upwards towards the proximal direction of the delivery device 30. FIGS. 17-18 show another optional tip orientation for a spiral anchor, which may comprise a spiral band 17. In some embodiments, the spiral anchor 15 may comprise a free end 22 which is angled or bent such that it points downwards towards the distal direction of the delivery device 30. Angling the free end 22 distally or proximally, or otherwise disposing the free end 22 away from the rest of the anchor 15 (for example radially outward therefrom), may aid in deployment of the anchor 15 from the delivery device and/or capture of the one or more structures (e.g., chordae tendineae, leaflets, etc.).

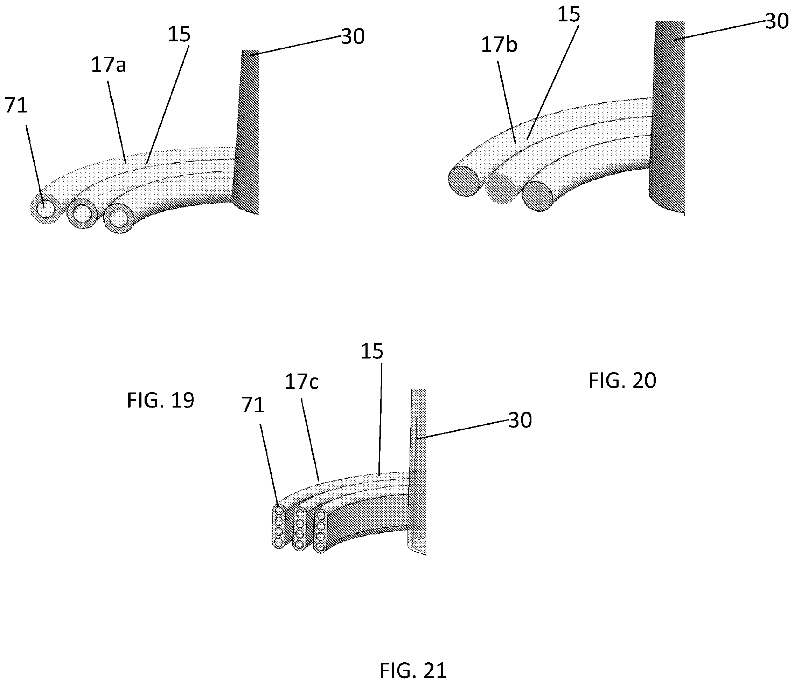

FIGS. 19-21 show various optional configurations for a spiral anchor, which can include any of the features described herein. FIG. 19 shows an exemplary anchor 15 comprising a spiral band 17a that is tubular (i.e., that has a hollow circular cross-section surrounding a lumen). The spiral anchor 15 may, for example, comprise a hypotube. The anchor 15 may be configured to pass another component (e.g. a wire, guidewire, etc.) therethrough (e.g., through the lumen 71 of the tubular band). FIG. 20 shows a spiral band 17b which may comprise a solid circular cross-section. The spiral band 17b may, for example, comprise a solid wire. FIG. 21 shows a spiral band 17c with a non-round, elongated cross-section (e.g., with a greater height in the axial direction than width in the radial direction). The band 17c can include a plurality of channels or lumens 71 disposed therein. Four channels are shown, but it will be understood to one of ordinary skill in the art that any number of channels may be utilized as desired. The lumens 71 may for example be left open or empty. Alternatively, or in combination, the lumens 71 may be filled, for example with one or more stiffening members. For example, at least one channel may be open and at least one channel may be filled.

The spiral anchor 15 may have a cross-section of any shape desired, for example a circular, tubular (e.g. hollow), square, elongated, triangular, or any other shaped cross-section. The spiral anchor 15 may comprise a solid anchor, or the spiral anchor may comprise one, two, three, four, five, or more lumens 71 or channels disposed therein. The lumens 71 or channels may be open and/or filled, for example with one or more stiffening members, guidewires, or the like. It will be understood by one of ordinary skill in the art that the spiral anchor may have any configuration, or combination of configurations, described herein or understood by one of ordinary skill in the art from the description herein.

For example, the one or more lumens 71 or channels may be configured to hold one or more stiffening members in order to provide structural support to the anchor 15. Alternatively, or in combination, a one or more guidewires may be passed through the one or more lumens 17 (e.g., the anchor 15 can be delivered to the heart over the one or more guidewires. The one or more guidewires can be translatably and removably disposed within the one or more lumens 71.

The spiral band 17 may have a cross-section of any shape desired, for example a circular, tubular (e.g. hollow), square, elongated, triangular, or any other shaped cross-section. The spiral band may comprise a solid band, or the spiral band may comprise one, two, three, four, five, or more lumens or channels disposed therein. The lumens or channels 71 may be open lumens and/or filled channels, for example with one or more stiffening members, guidewires, or the like. It will be understood by one of ordinary skill in the art that the spiral band may have any configuration, or combination of configurations, described herein or understood by one of ordinary skill in the art from the description herein.

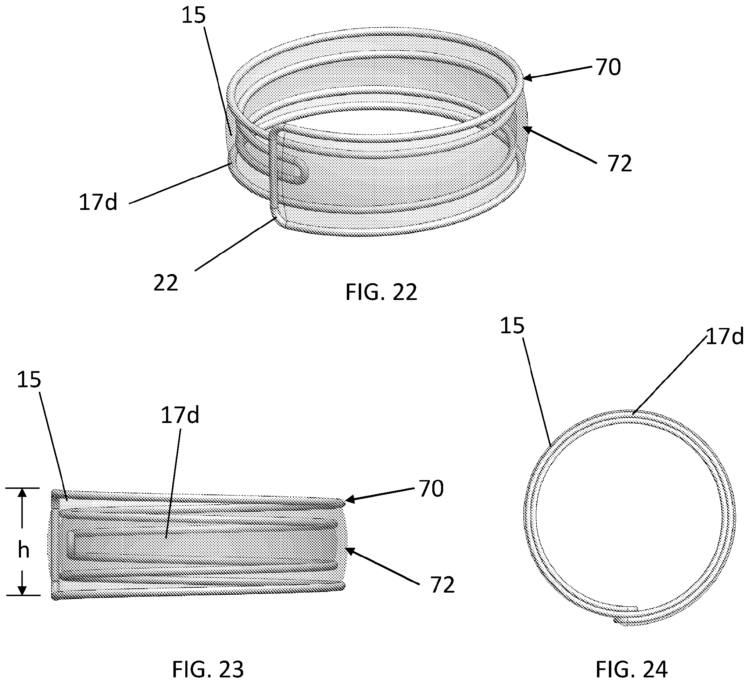

FIGS. 22-24 show several views of another exemplary anchor 15 comprising a spiral band 17d. The spiral band 17d may be a tapered spiral band. The tapered spiral band 17d may be deployed and configured to anchor a frame structure of a valve prosthesis adjacent a native valve as described herein. The anchor 15 may, for example, be configured to be fully advanced from a first side of a native valve in a patient (e.g. an atrial side) to a second side of the native valve (e.g. into a ventricle of the heart) and anchor a frame structure to the native valve when the frame structure is in an expanded configuration adjacent the native valve as described herein. The tapered spiral band 17d may comprise a delivery (e.g., elongated) configuration and a deployed configuration (and optional intermediate configurations) substantially similar to those shown in FIGS. 4-69. The tapered spiral band 17d may be configured to be actuated from the elongated configuration to the deployed configuration adjacent a native valve in a patient. The tapered spiral band 17d may be delivered to the native valve by a delivery device as described herein. The tapered spiral band 17d may be deployed adjacent the native valve substantially similarly to other anchor embodiments as described herein. In some embodiments, the anchor 15 may comprise one or more locking mechanisms configured to maintain the anchor 15 in the deployed configuration. The one or more locking mechanisms may be any of the locking mechanisms described herein or understood by one of ordinary skill in the art from the description herein.

The anchor 15 may comprise a tapered spiral band 17d having a free end 22. The other end of the tapered spiral band 17d may be coupled to the top (proximal end) or bottom (distal end) of a frame structure as described herein. Alternatively, or in combination, the other end of the tapered spiral band 17d may not be attached to a frame structure as described herein. The free end 22 of the tapered spiral band 17d may facilitate capturing of the valve leaflets and/or chordal tendineae within the sweep of the free end 22 during rotation as described herein. During rotation of the tapered spiral band 17d, the leaflets and/or chordae tendineae may be captured by the free end 22 and trapped between the valve frame structure and an interior surface of the spiral band 17d.

The free end 22 of the spiral band 17d may be sized and dimensioned for insertion through the native valve, for example through tissue at or near a commissure of the native valve or through the valve opening itself. In some embodiments, the free end 22 may comprise an atraumatic tip to avoid reduce risk of injury to the native valve tissue and leaflets. For example, the free end may comprise a blunt end, a ball tip, a curved tip (e.g. J-tip or pigtail), or other atraumatic shapes. Alternatively, the free end 22 may be configured for piercing tissue. In various embodiments, the free end 22 is separate from and extends outward from the main coils of the anchor 15. In various embodiments, the main body coils of the anchor 15 circumscribe an area (in the case of a spiral coil) or a volume (in the case of a helical coil) having a diameter, and the free end 22 extends to a radius greater than the diameter of the anchor 15. In various embodiments, the free end 22 extends to a radius substantially greater than the diameter of the anchor 15. In various embodiments, the free end 22 is configured to circumscribe a larger diameter than the anchor 15. In various embodiments, the free end 22 is configured to circumscribe all of the chordae tendineae of the native valve to be treated.

The free end 22 of the tapered spiral band 17d may optionally be rotated around one or more structures on the second side of the native valve such that the one or more structures (e.g., chordae, leaflets, or annulus) are pulled radially inwards towards the longitudinal axis of the anchor 15 and/or towards the longitudinal axis of the delivery device 30. The spiral band 17d and/or free end 22 may be configured such that minimal torque is applied to the one or more structures. Alternatively, or in combination, the tapered spiral band 17d and/or free end 22 may be configured such that the one or more structures are not rotated, or are minimally rotated, during rotation of the spiral band 17d. For example, the spiral band 17d may comprise one or more spaces between loops of the spiral band (for example, spaces 18 shown in FIG. 9) which facilitate movement of the captured tissue (e.g. chordae and/or leaflets) from the free end 22 to the center of the spiral structure with little or no torque and/or rotation of the structures during rotation of the tapered spiral band 17d as described herein. Alternatively, or in combination, the spiral band 17d may be configured such that, when fully deployed (for example, as shown in FIG. 24), none of the structures reside between the loops of the spiral. Instead, the one or more structures may sit radially inward of the loops in order to facilitate capture of the one or more structures between the spiral band 17d and the expanded frame structure as described herein. The one or more structures may retain or nearly retain their normal anatomical position when the spiral band 17d is fully deployed.

Spiral band 17d may be configured to taper in height (h) axially. For example, spiral band 17d may be configured to taper in height from a first end of the spiral band 17d ( which may be coupled to a delivery device and/or frame structure as described herein) to a free end 22 of the spiral band 17d. In some embodiments, the spiral band 17d may taper in height from a proximal end to a distal end. Alternatively, the spiral band 17d may taper in height from a distal end to a proximal end.

As shown best in FIG. 23, the spiral band 17d may be tapered such that subsequent turns of the tapered spiral band 17d nest into each other so as to reduce a radial footprint of the tapered spiral band.

The spiral band 17d may, for example, comprise a solid wire-like spiral band similar to that shown in FIG. 16, but with a taper as described herein. In some embodiments, the spiral band 17d may comprise a plurality of spiral support wires.

Alternatively, or in combination, at least a portion of the tapered spiral band may comprise a support structure 70 and a band material 72 disposed therein (or thereon as desired by one of ordinary skill in the art).

The support structure 70 may, for example, comprise a wire. The support structure 70 may be formed of a material having sufficient rigidity to hold a predetermined shape. The support structure 70 may, for example, be formed of a shape memory material (e.g. NiTi). It may be desirable for at least an end portion (e.g. free end 22) to be relatively rigid such that it can exert a force to move chordal tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

The band material 72 may comprise a permeable, semi-permeable, or impermeable material. The band material 72 may be flexible, semi-flexible, or rigid. In some embodiments, the band material 72 may be relatively soft so as to reduce the risk of injury to the one or more structures during rotation of the tapered spiral band 17d. The band material 72 may, for example, comprise a webbing material, a fabric, a polymeric material, an elastomeric material, or the like. The band material 72 may span the structural support 70 so as to reduce leakage therethrough. Alternatively, or in combination, the band material 72 may be configured to improve alignment of the support structure 70 wire.

It will be understood by one of ordinary skill in the art from the description herein that any of the anchors 15 described herein may comprise a support structure and a material disposed therein or thereon as described with referenced to tapered spiral band 17d.

The tapered spiral band 17d may comprise one or more loops. For example, the spiral band 17d may comprise a plurality of loops, which may increase the radial strength of the anchor by increasing friction and addition structural support. The one or more loops of the spiral band 17d may spiral radially outward from a central point or central axis of the spiral, for example along an axis which is coaxial with a longitudinal axis of a delivery device 30 such that the spiral band 17d lies approximately along a plane perpendicular to the longitudinal axis of a delivery device.

In some embodiments, the one or more loops of the spiral band 17d may comprise one or more spaces therebetween, which may be substantially similar to spaces 18 shown in FIG. 9. The spaces may facilitate movement of the captured tissue (e.g. chordae and/or leaflets) from the free end 22 to the center of the spiral structure during rotation of the anchor 15 as described herein.

In some embodiments, the support structure 70 may comprise one or more channels or lumens 71 disposed therein. The support structure 70 may comprise a hollow, tubular cross-section. The support structure 70 may, for example, comprise a hypotube. The lumen of the support structure 70 may be configured to pass another component (e.g. a wire, guidewire, etc.) therethrough.

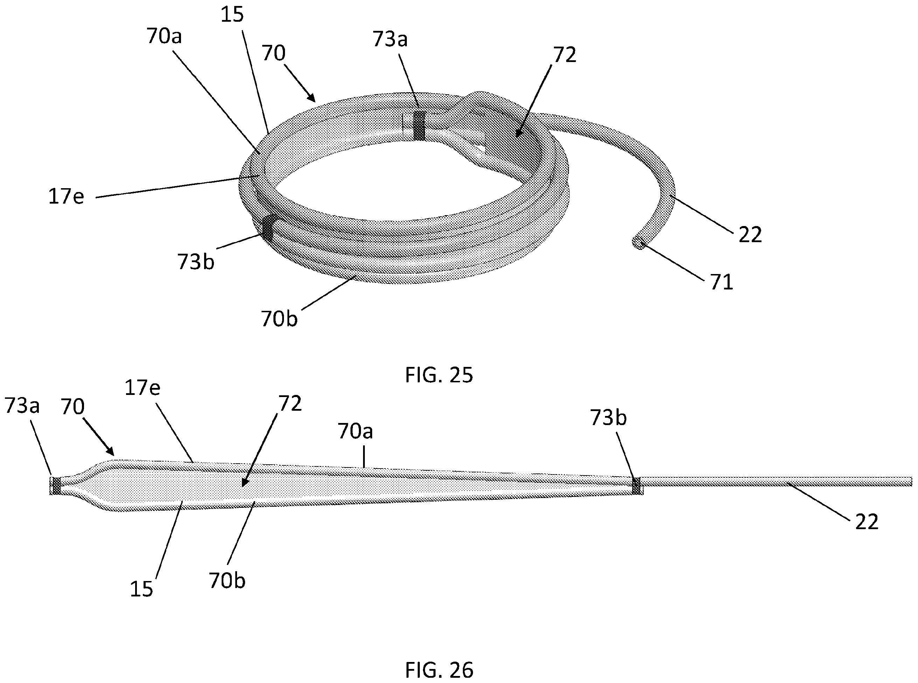

FIGS. 25-28 show several views of another anchor 15 comprising a tapered spiral band 17e. FIGS. 25, 27, and 28 show the anchor 15 in a deployed configuration. FIG. 26 shows the anchor 15 in an elongated delivery configuration. The spiral band 17e may be substantially similar to tapered spiral band 17d shown in FIGS. 22-24 except that the support structure 70 may comprise more than one wire. The support structure 70 may, for example, comprise an upper wire 70a and a lower wire 70b. The wires 70a, 70b may be coupled to one another at a proximal attachment point 73a and/or a distal attachment point 73b. The wires 70a, 70b may be round wires or have other cross-sectional shapes. In various embodiments, the support structure 70 may comprise a scaffold such as a laser-etched Nitinol scaffold or a mesh. The spiral band 17e may be tapered such that subsequent turns of the tapered spiral band 17e nest into each other so as to reduce a radial footprint of the tapered spiral band. Wires 70a, 70b may be configured to nest with one another when the in the deployed configuration.

The tapered spiral band 17e may comprise a delivery (e.g., elongated) configuration and a deployed configuration (and optional intermediate configurations) as described herein. The tapered spiral band 17e may be configured to be actuated from the elongated configuration to the deployed configuration adjacent a native valve in a patient. The tapered spiral band 17e may be delivered to the native valve by a delivery device in the elongated configuration as described herein. The tapered spiral band 17e may be coupled to the delivery device and/or a frame structure of a valve prosthesis as described herein, for example at a proximal portion (e.g. adjacent proximal attachment point 73a on an interior of the spiral) or distal portion (e.g. adjacent distal attachment point 73b on an exterior of the spiral) thereof. The tapered spiral band 17e may be deployed adjacent the native valve substantially similarly to other anchor embodiments as described herein.

In some embodiments, the upper wire 70a and lower wire 70b may be bundled together during deployment from an undeployed configuration to an intermediate deployed configuration. The intermediate configuration may be configured to reduce the size the size of the lumen and/or aperture in or through which, respectively, the spiral anchor 15 may travel (for example, a lumen or aperture of the delivery device) during deployment. The anchor 15 may be maintained in the intermediate configuration during rotation around the one or more structures as described herein. In at least some instances, coupling the wires 70a, 70b together into an intermediate configuration may facilitate alignment of the wires making up the support structure 70. After delivery from the delivery device and/or rotation around one or more structures, the anchor 15 may be fully deployed into the deployed configuration by releasing the bundle and allowing the wires 70a, 70b to "spring out" into the deployed configuration.

The support structure 70 may have a band material 72 disposed therein or thereon as described herein. The band material 72 may span the distance between wires 70a, 70b in order to couple the wires 70a, 70b to one another. The band material 72 may span the structural support 70 so as to reduce leakage therethrough as described herein. Alternatively, or in combination, the band material 72 may be configured to improve alignment of the support structure 70 wires 70a, 70b such that they maintain a desired relative position to one another.

The anchor 15 may comprise a free end 22. The free end 22 may be substantially similar to any of free ends described herein. The other end of the tapered spiral band 17e may be coupled to the top (proximal end) or bottom (distal end) of a frame structure as described herein. Alternatively, or in combination, the other end of the tapered spiral band 17e may not be attached to a frame structure as described herein. The free end 22 of the tapered spiral band 17e may facilitate capturing of the valve leaflets and/or chordal tendineae within the sweep of the free end 22 during rotation as described herein. During rotation of the tapered spiral band 17e, the leaflets and/or chordae tendineae may be captured by the free end 22 and trapped between the valve frame structure and an interior surface of the spiral band 17e against the support structure 70 and/or band material 72.

The free end 22 of the spiral band 17e may be sized and dimensioned for insertion through the native valve, for example through tissue at or near a commissure of the native valve or through the valve opening itself. In some embodiments, the free end 22 may comprise an atraumatic tip to avoid reduce risk of injury to the native valve tissue and leaflets. For example, the free end may comprise a blunt end, a ball tip, a curved tip (e.g. J-tip or pigtail), or other atraumatic shapes. Alternatively, the free end 22 may be configured for piercing tissue. In various embodiments, the free end 22 is separate from and extends outward from the main coils of the anchor 15. In various embodiments, the main body coils of the anchor 15 circumscribe an area (in the case of a spiral coil) or a volume (in the case of a helical coil) having a diameter, and the free end 22 extends to a radius greater than the diameter of the anchor 15. In various embodiments, the free end 22 extends to a radius substantially greater than the diameter of the anchor 15. In various embodiments, the free end 22 is configured to circumscribe a larger diameter than the anchor 15. In various embodiments, the free end 22 is configured to circumscribe all of the chordae tendineae of the native valve to be treated. In various embodiments, the free end 22 may be shaped and configured to reduce the risk of counterrotation. For example, the tip 22 may have a curled end to cause the free end 22 to snag chordae if it is rotated in a direction opposite the anchoring rotation.

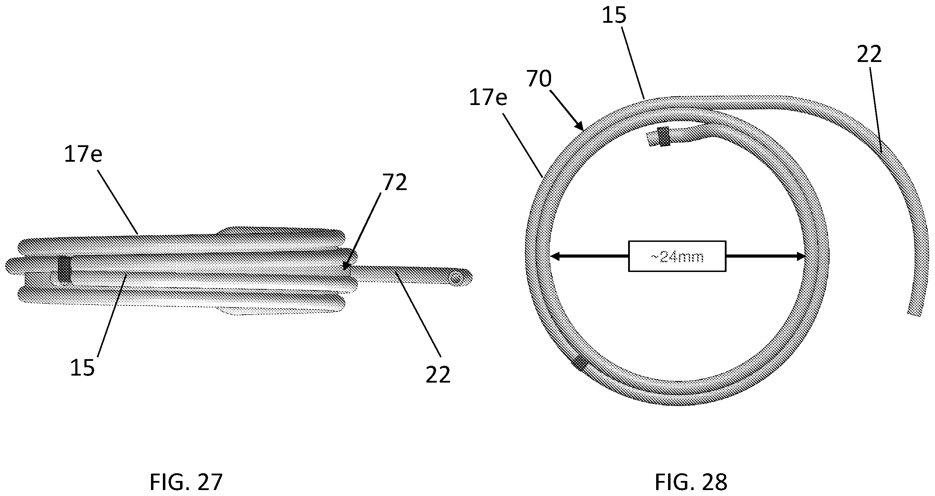

The free end 22 of the tapered spiral band 17e may optionally rotated around one or more structures on the second side of the native valve such that the one or more structures (e.g., chordae, leaflets, or annulus) are pulled radially inwards towards the longitudinal axis of the anchor 15 and/or towards the longitudinal axis of the delivery device 30. The spiral band 17e and/or free end 22 may be configured such that minimal torque is applied to the one or more structures. Alternatively, or in combination, the tapered spiral band 17e and/or free end 22 may be configured such that the one or more structures are not rotated, or are minimally rotated, during rotation of the spiral band 17e. For example, the spiral band 17e may comprise one or more spaces between loops of the spiral band (for example, spaces 18 shown in FIG. 9) which facilitate movement of the captured tissue (e.g. chordae and/or leaflets) from the free end 22 to the center of the spiral structure with little or no torque and/or rotation of the structures during rotation of the tapered spiral band 17e as described herein. Alternatively, or in combination, the spiral band 17e may be configured such that, when fully deployed (for example, as shown in FIG. 28), none of the structures reside between the loops of the spiral. Instead, the one or more structures may sit radially inward of the loops in order to facilitate capture of the one or more structures between the spiral band 17e and the expanded frame structure as described herein. The one or more structures may retain or nearly retain their normal anatomical position when the spiral band 17e is fully deployed.

The free end 22 may be disposed radially outwards from the support structure 70. Disposing the free end 22 radially outward from the remainder of support structure 70 may, for example, aid in deployment of the anchor 15 from the delivery device and/or capture of the one or more structures as described herein.

The free end 22 may be angled proximally (e.g., towards a proximal portion of the anchor 15 and a distal end of the delivery device 30) or distally (e.g., away from a proximal portion of the anchor 15 and towards a proximal portion of the delivery device 30) from the support structure 70. Angling the free end 22 proximally or distally towards or away from the delivery device may, for example, aid in deployment of the anchor 15 from the delivery device and/or capture of the one or more structures as described herein.

In some embodiments, the support structure 70 may comprise one or more channels or lumens 71 disposed therein. For example, one or more of the wires 70a, 70b may comprise one or more channels or lumens 71. One or more of the wires 70a, 70b may comprise a hollow, tubular cross-section. The spiral band 17e may, for example, comprise a hypotube. The lumen of the spiral band 17e may be configured to pass another component (e.g. a wire, guidewire, etc.) therethrough. The channels or lumens 71 may for example be left as open lumens. Alternatively, or in combination, the channels or lumens 71 may be filled, for example with one or more stiffening members.

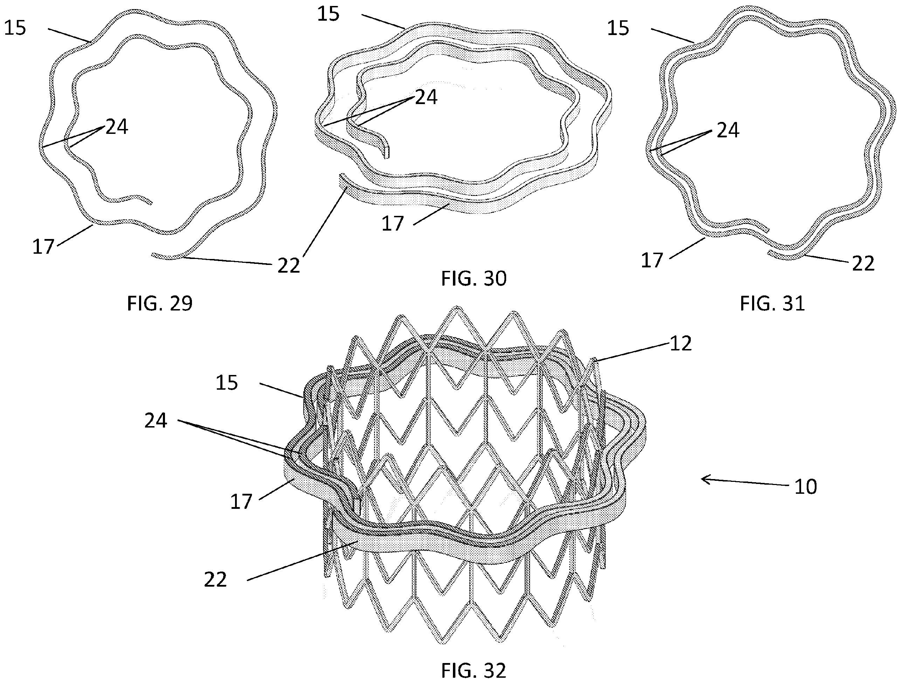

FIGS. 29-32 show several views of an optional locking mechanism for a flat spiral anchor. FIG. 29 shows a top view of a flat spiral anchor 15 comprising a spiral band 17 having a plurality of interlocking features 24 disposed thereon in an intermediate deployed or undeployed configuration (which may be substantially similar to the configurations shown in FIGS. 10-11 and described herein). FIG. 30 shows a perspective view of the spiral band anchor 15 in the undeployed configuration. FIG. 31 shows a top view of the spiral band anchor in a fully deployed configuration. FIG. 32 shows a perspective view of a valve prosthesis 10 comprising the spiral band anchor 15 in the deployed configuration disposed around a frame structure 12 in an expanded configuration.

The valve prosthesis 10 may be substantially similar to any of the valve prostheses described herein. The frame structure 12 may have an unexpanded configuration (for example, a compressed configuration as described herein) and an expanded configuration as described herein. The frame structure 12 may be substantially similar to any of the frame structures described herein or understood by one of ordinary skill in the art from the description herein. The anchor 15 may comprise a spiral band 17. The anchor 15 may be configured to anchor the frame structure 12 to the native valve when the frame structure 12 is in the expanded configuration adjacent the native valve. The frame structure 12 may be configured to be actuated from the unexpanded configuration to the expanded configuration adjacent a native valve in a patient. The spiral band anchor 15 may be deployed and configured to anchor the frame structure 12 of the valve prosthesis 10 adjacent a native valve as described herein. The anchor 15 may, for example, be configured to be fully advanced from a first side of a native valve in a patient (e.g. an atrial side) to a second side of the native valve (e.g. into a ventricle of the heart) and anchor the frame structure 12 to the native valve when the frame structure 12 is in an expanded configuration adjacent the native valve as described herein. The spiral band anchor 15 may be delivered to the native valve by a delivery device as described herein.

The anchor 15 may comprise a spiral band 17 having a free end 22. The spiral band 17 may be substantially similar to any of the spiral bands described herein. The free end 22 may be substantially similar to the free end of other anchors described herein. The other end of the anchor 15 (e.g., spiral band 17) may be coupled to the top (proximal end), bottom (distal end), or an intermediate portion (e.g. middle) of the frame structure 12 as described herein. Alternatively, or in combination, the other end of the anchor 15 may not be attached to the frame structure 12 as described herein. The free end 22 of the spiral anchor 15 may facilitate capturing of the valve leaflets and/or chordae tendineae within the sweep of the free end during rotation as described herein. During rotation of the spiral anchor 15, the leaflets and/or chordae tendineae may be captured by the free end 22 and trapped between the valve frame structure 12 and an interior surface of the spiral anchor 15. The anchor 15 may comprise one or more spaces between loops of the anchor 15 which facilitate movement of the captured tissue (e.g. chordae and/or leaflets) from the free end 22 to the center of the spiral structure with little or no torque and/or rotation of the structures during rotation of the anchor 15 as described herein. At least a portion of the spiral anchor 15 may be formed of a material having sufficient rigidity to hold a predetermined shape as described herein.

In some embodiments, the anchor 15 may comprise a delivery (e.g., elongated) configuration when disposed within a delivery device as described herein. The delivery device may be configured to deploy the anchor 15 from the delivery configuration to an undeployed or first intermediate deployed configuration as described herein (as shown in FIGS. 29 and 30). The anchor 15 may then be actuated into the deployed or second fully deployed configuration (as shown in FIGS. 31 and 32), for example by expanding the frame structure 12 within the anchor 15 or by rotating the anchor 15 to capture the one or more structures as described herein. In some embodiments, the anchor 15 may be configured to be actuated into more than one intermediate deployed configuration, for example, by partial expansion of the frame structure 12.

The anchor 15 may comprise a plurality of loops of a spiral as described herein. The anchor 15 may comprise a more compact spiral, with more loops, in the undeployed or first intermediate deployed configuration compared to the deployed or second fully deployed configuration. As the anchor 15 expands the spiral loops may unwind. In some embodiments, the outer perimeter of the anchor 15 may be substantially similar in the undeployed (or first intermediate deployed) and deployed (or second fully deployed) configurations. As the spiral loops unwind, the spiral may expand from the center, for example simultaneously with expansion of the frame structure in the center of anchor 15, such that the spiral loops are expanded against one another as shown in FIG. 31. As will be understood by one of ordinary skill in the art, the shape, size, and number of loops may determine the limits of expansion of the anchor 15 and alteration of any or all of these parameters may be done to generate a final deployed configuration desired from an initial undeployed configuration.

The anchor 15 may have a generally spiral shape in the deployed configuration. The anchor 15 may be formed as a flat spiral (in the deployed configuration) whereby the loops generally are positioned within the same plane (the plane being perpendicular to a longitudinal axis). The plurality of loops of the anchor 15 may be spiral radially outward from a central point or central axis of the spiral, for example along an axis which is coaxial with a longitudinal axis of a delivery device such that the anchor 15 lies approximately along a plane perpendicular to the longitudinal axis of the delivery device as described herein. The plurality of loops may comprise at least 360 degrees of rotation when deployed such that the loops wrap around one another and provide additional mechanical leverage against the frame structure in order to facilitate anchoring of the frame structure as described herein. Additional loops or partial loops may provide additional mechanical strength and/or leverage.

The spiral band anchor 15 may be substantially similar to any of the anchors described herein with the addition of interlocking features 24 disposed along the loops of the anchor 15 (e.g., along the loops of spiral band 17) such that they interlock when expanded against one another. The interlocking features 24 may, for example, comprise correspondingly-shaped waves, bends, humps, or the like disposed on adjacent loops of the spiral band anchor 15 such that the interlocking features 24 on adjacent loops may be nested with each other when the anchor 15 is in the deployed configuration. The interlocking features can provide a locking mechanism in the general sense. In some embodiments, the interlocking features may act more like index features for the opening and/or closing of the spiral band (i.e., expansion and/or contraction of the diameter). In this manner, the interlocking features may index the opening and/or closing by discreet increments. In some embodiments, the interlocking features may positively control the opening and/or closing of the spiral band. One will appreciate from the description herein that the resistance provided by the interlocking features may be adjusted depending on the application. In some embodiments, the resistance may be relatively high to prevent or reduce the risk of the diameter of the spiral anchor or band changing diameter. One will appreciate that the interlocking features may be designed and configured in various other manner. Although shown as a somewhat repeating pattern in FIGS. 29-32, the interlocking features may have one or more shapes and sizes. The pattern may be repeating or non-repeating. The spiral anchor or band may include different sets of interlocking features. For example, it may be desirable to provide one or more sets of interlocking features to index self-assembly and/or anchoring of the spiral anchor or band to the chordae and another set(s) configured to prevent the spiral anchor or band from expanding beyond a certain predetermined diameter, such as to increase the opposing force against the expanding frame.

In some embodiments, the spiral anchor 15 may comprise a more than one pair of interlockingly-shaped features 24 disposed on adjacent loops of the anchor 15 (e.g., on adjacent loops of the spiral band 17) in order to ensure secure locking and/or to provide for a plurality of intermediate expanded/deployed configurations between the fully undeployed and the fully deployed configuration. For example, the spiral band anchor 15 may comprise a plurality of waves 24 in adjacent loops of the generally spiral-shaped anchor 15 which can provide intermittent locking of the anchor 15 as the anchor 15 is deployed in multiple configurations. As the anchor 15 is expanded, the waves 24 may move into and out of nesting with one another, locking when nested together and expanding when enough energy has been applied to disengage the nested waves from one another.

In some embodiments, the plurality of features 24 may be disposed along the generally spiral-shaped anchor 15 at pre-determined distances. In some embodiments, the plurality of features 24 may be disposed along the generally spiral-shaped anchor 15 at regular intervals. In some embodiments, the plurality of features 24 may be disposed along only a portion of the anchor 15. In some embodiments, the plurality of features 24 may be disposed along the entire anchor 15.

In some embodiments, the plurality of interlocking features 24 may comprise two, four, six, eight, ten, twelve, fourteen, sixteen, eighteen, twenty, or more features 24 disposed on adjacent loops of the spiral band 17. For example, each adjacent loop may comprise one, two, three, four, five, six, seven, eight, nine, ten, or more features 24 per loop.

In some embodiments, the plurality of features 24 may comprise a plurality of waves disposed along the generally spiral-shaped anchor 15 such that the anchor resembles a flower-like shape (e.g., as shown in FIGS. 29-32).

It will be understood by one or ordinary skill in the art that any of the anchors described herein may comprise any of the locking mechanisms, or any combination of locking mechanisms, described herein or understood based on the teachings herein.

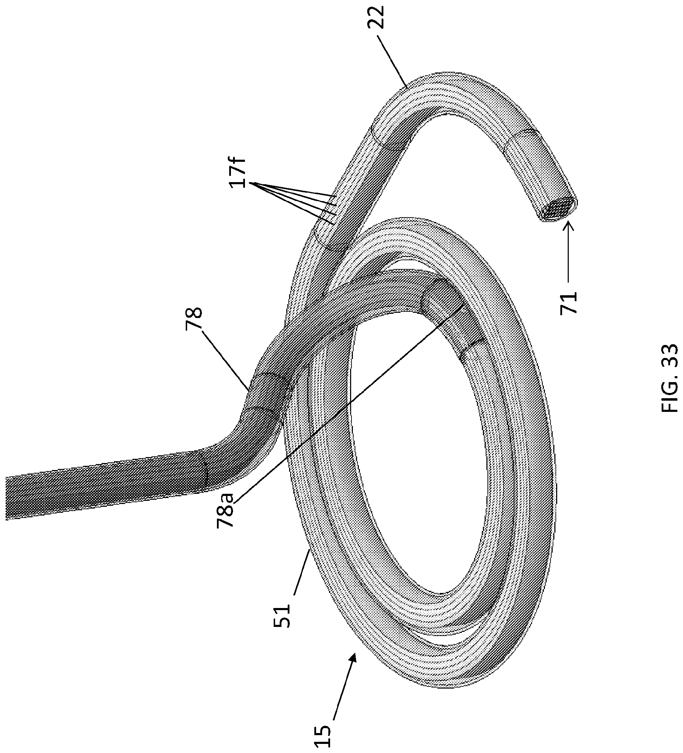

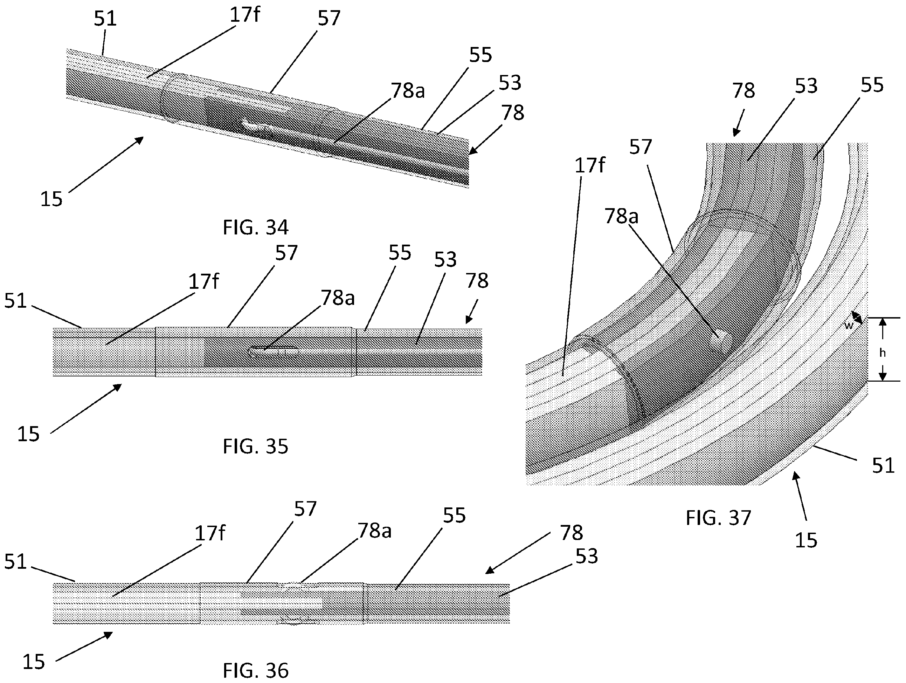

FIGS. 33-37 show several views of a multi-ply anchor 15 that can be used as part of any of the valve prostheses described herein. FIG. 33 shows a perspective view of a flat spiral anchor 15 comprising a plurality of spiral bands 17f disposed within a lumen 71 of the anchor 15. The anchor 15 is in a deployed configuration. FIG. 34 shows a perspective view of a connection point between the multi-ply anchor 15 and a proximal pusher 78 (otherwise called an attachment arm) in an undeployed (e.g., elongated) configuration. FIG. 35 shows a side view of the connection point in the undeployed configuration. FIG. 36 shows a top view of the connection point in the undeployed configuration. FIG. 37 shows a perspective view of the connection point in the deployed configuration. The anchor 15 may be part of any of the valve prostheses described herein and may be used to anchor any of the frame structures described herein.

The anchor 15 comprises a housing 51 having a lumen 71. A plurality of spiral bands 17f (also referred to herein as "ply"s) can be positioned adjacent to one another within the lumen 71 (e.g., stacked radially relative to one another). Each of the plurality of spiral bands 17f may be substantially similar to any of the spiral bands 17f described herein. The plurality of spiral bands 17f may be disposed within a single lumen 71 of the anchor 15. The housing 51 of the anchor 15 may act as a covering to keep the plurality of spiral bands 17f together, adjacent to, and/or aligned with one another. In some embodiments, the housing 51 may comprise a polymer material, such as Polyethylene terephthalate or a thermoset polymer like polyurethane. In some embodiments, an outer surface of the housing 51 may be coated with one or more substances or drugs, for example heparin.

The plurality of spiral bands 17f are configured to move relative to one another. In some embodiments, the plurality of spiral bands 17f are slidably or translatably disposed next to one another within the lumen 71 of the anchor 15. For example, the plurality of spiral bands 17f may be configured to move transversely relative to one another. Relative movement between the spiral bands 17f may enable greater flexibility of motion of the anchor 15 during winding, unwinding, bending, or otherwise contorting during deployment and/or use. In addition to providing greater flexibility compared to a single thick spiral band, having the plurality of spiral bands 17f slidable relative to one another can improve stiffness and strength of the anchor 15 without sacrificing much maneuverability compared to a single thin band or wire. In some embodiments, the spiral bands 17f may move relative to one another only deployment and/or positioning of the anchor 15 and can then be locked relative to one another once fully deployed. In some embodiments, the spiral bands 17f can be unlocked relative to one another after locking (e.g., to enable flexibility during repositioning of the anchor 15).

The plurality of spiral bands 17f may comprise one or more loops. For example, the anchor 15 may comprise a plurality of loops (with the spiral bands 17f extending therein), which may increase the radial strength of the anchor by increasing friction and addition structural support.

The plurality of spiral bands 17f may be formed of a material having sufficient rigidity to hold a predetermined shape. The spiral bands 17f may, for example, be formed of a shape memory material (e.g. NiTi). The shape memory material may advantageously enable formation of the bands 17f into a complex shape via heating setting. Additionally, the bands 17f of shape memory material may exhibit super-elastic properties, which can advantageously help enable introduction of the anchor 15 into a delivery catheter and/or deployment of the anchor 15 into a preset shape. The relative rigidity and/or local stiffness of the anchor 15 can be adjusted as described herein including, but not limited to, changing the shape of the spiral bands 17f and/or modifying connections between the spiral bands 17f to allow for more or less relative movement. It may be desirable for at least an end portion (e.g. free end 22) of the anchor 15 to be relatively rigid such that it can exert a force to move chordae tendineae, while still retaining flexibility to be collapsed within a delivery device. In various embodiments, the end portion of the anchor 15 only needs sufficient rigidity to hold its shape and will deform under a load. For example, the end portion of the anchor 15 may be configured with a similar rigidity to a guidewire or slightly stiffer.

In some embodiments, the anchor 15 housing 51 may comprise a hollow, tubular cross-section. The spiral anchor 15 may, for example, comprise a hypotube. The lumen 71 of the housing 51 may be configured to pass the plurality of spiral bands 17f therethrough (i.e., the hold the spiral bands 17f therein). The anchor 15 comprises a flat spiral or planar spiral shape in the deployed configuration.

The plurality of spiral bands 17f may each comprise a flat spiral or planar spiral shape in the deployed configuration. The spiral bands 17f may have a cross-section of any shape desired, for example a circular, tubular (e.g. hollow), square, elongated, triangular, or any other shaped cross-section. In some embodiments, the spiral bands 17f may have an elongated sheet-like cross-section (i.e., with a greater height h than width w). In some embodiments, the surfaces of the spiral bands 17f that engage one another can be flat, which may enable better stacking and transverse movement of the spiral bands 17f relative to one another. When placed next to one another inside the lumen 71 of the anchor, the plurality of spiral bands 17f together may have a rectangular or square cross-section. For example, a plurality of spiral bands 17f having a square cross-section when together may be disposed within a lumen 71 having a circular cross-section in order to improve ventricular stability of the anchor 15 after deployment.

The anchor 15 is configured to anchor the frame structure 12 to the native valve when the frame structure 12 is in the expanded configuration adjacent the native valve. The frame structure 12 may be configured to be actuated from the unexpanded configuration to the expanded configuration adjacent a native valve in a patient. The spiral bands 17f may provide enhanced flexibility to the anchor 15 to enable the anchor 15 to easily move from the unexpanded configuration to the expanded configuration and vice versa. The anchor 15 may be deployed and configured to anchor the frame structure 12 of the valve prosthesis 10 adjacent a native valve as described herein. The anchor 15 may, for example, be configured to be fully advanced from a first side of a native valve in a patient (e.g. an atrial side) to a second side of the native valve (e.g. into a ventricle of the heart) and anchor the frame structure 12 to the native valve when the frame structure 12 is in an expanded configuration adjacent the native valve as described herein. The spiral band anchor 15 may be delivered to the native valve by a delivery device as described herein.

The free end 22 may be substantially similar to the free end of other anchors described herein. The other end of the anchor 15 may be coupled to the top (proximal end), bottom (distal end), or an intermediate portion (e.g. middle) of the frame structure 12 as described herein. Alternatively, or in combination, the other end of the anchor 15 may not be attached to the frame structure 12 as described herein. The free end 22 of the spiral anchor 15 may facilitate capturing of the valve leaflets and/or chordae tendineae within the sweep of the free end during rotation as described herein. During rotation of the spiral anchor 15, the leaflets and/or chordae tendineae may be captured by the free end 22 and trapped between the valve frame structure 12 and an interior surface of the spiral anchor 15. The anchor 15 may comprise one or more spaces between loops of the anchor 15 which facilitate movement of the captured tissue (e.g. chordae and/or leaflets) from the free end 22 to the center of the spiral structure with little or no torque and/or rotation of the structures during rotation of the anchor 15 as described herein. At least a portion of the spiral anchor 15 may be formed of a material having sufficient rigidity to hold a predetermined shape as described herein.

In some embodiments, the free end 22 may be disposed radially outward from the body of the anchor 15 to form a grabber arm as described herein. The free end 22 of the anchor 15 may extend radially outward from the frame 12, and in particular from the remainder of the anchor 15. The free end 22 may be "L"-shaped in some embodiments in order to facilitate encircling of the chordae tendineae while reducing the likelihood of the anchor 15 hitting and damaging the ventricular wall. The l-shaped free end 22 may also be configured to guide the chordae tendineae into the loops of the anchor 15 during rotation of the anchor 15 as described herein.

In some embodiments, the anchor 15 may comprise a delivery (e.g., elongated) configuration when disposed within a delivery device as described herein. The delivery device may be configured to deploy the anchor 15 from the delivery configuration to an undeployed or first intermediate deployed configuration as described herein. The anchor 15 may then be actuated into the deployed or second fully deployed configuration, for example by expanding the frame structure 12 within the anchor 15 or by rotating the anchor 15 to capture the one or more structures as described herein. In some embodiments, the anchor 15 may be configured to be actuated into more than one intermediate deployed configuration, for example, by partial expansion of the frame structure 12.

The anchor 15 may comprise a plurality of loops of a spiral as described herein. The anchor 15 may comprise a more compact spiral, with more loops, in the undeployed or first intermediate deployed configuration compared to the deployed or second fully deployed configuration. As the anchor 15 expands, the spiral loops may unwind (e.g., causing the spiral bands 17f to slide relative to one another). In some embodiments, the outer perimeter of the anchor 15 may be substantially similar in the undeployed (or first intermediate deployed) and deployed (or second fully deployed) configurations. As the spiral loops unwind, the spiral may expand from the center, for example simultaneously with expansion of the frame structure in the center of anchor 15, such that the spiral loops are expanded against one another. As will be understood by one of ordinary skill in the art, the shape, size, and number of loops may determine the limits of expansion of the anchor 15 and alteration of any or all of these parameters may be done to generate a final deployed configuration desired from an initial undeployed configuration.

The anchor 15 may have a generally spiral shape in the deployed configuration. The anchor 15 is formed as a flat spiral (in the deployed configuration) whereby the loops generally are positioned within the same plane (the plane being perpendicular to a longitudinal axis). The plurality of loops of the anchor 15 may be spiral radially outward from a central point or central axis of the spiral, for example along an axis which is coaxial with a longitudinal axis of a delivery device such that the anchor 15 lies approximately along a plane perpendicular to the longitudinal axis of the delivery device as described herein. The plurality of loops may comprise at least 360 degrees of rotation when deployed such that the loops wrap around one another and provide additional mechanical leverage against the frame structure in order to facilitate anchoring of the frame structure as described herein. Additional loops or partial loops may provide additional mechanical strength and/or leverage.

As shown best in the close-ups of FIGS. 34-37, during deployment, the anchor 15 may be coupled to a proximal pusher 78. A proximal end of the anchor 15 may be detachably coupled to the proximal pusher 78. The proximal pusher 78 may be disposed within a lumen of the delivery device and may operably couple the anchor 15 to the delivery device. The proximal pusher 78 may act as an actuation mechanism and facilitate longitudinal translation of the anchor 15 through the delivery device during deployment. For example, a proximal end of the proximal pusher 78 may be coupled to an actuation mechanism in/on the delivery device in order to translate the anchor 15. Alternatively, the proximal end of the proximal pusher 78 may be manually manipulated by, at, or near a proximal end of the delivery device in order to translate the anchor 15.

The proximal pusher 78 may include a stack of flat wires 53 (e.g., arranged as plys) housed in a tubular housing 55. The flat wires 53 may be stacked adjacent to one another in the radial direction (i.e., relative to the spiral of the anchor 15). The housing 55 may, for example, comprise a mesh. The mesh may comprise a wire or polymer mesh. A distal end of the proximal pusher78 may comprise a hypotube 57 bonded to the housing 55. The hypotube 57 may provide additional support to the connection point between the anchor 15 and the pusher arm 78.

The stack of flat wires 53 of the proximal pusher 78 may be substantially similar in cross-section and structure to the plurality of spiral bands 17f. For example, each of the flat wires 53 can be aligned with and/or substantially continuous with each of the spiral bands 17f when the anchor 15 is connected to the proximal pusher 78. In some embodiments, and as shown in FIGS. 34, 36, and 37, the outermost two flat wires 53 can extend further distally than the innermost flat wires 53. Correspondingly, the innermost spiral bands 17f in lumen 71 can extend further proximally than the outermost two spiral bands 17f. The differing lengths of the wires 53 and bands 17f can help ensure that the pusher arm 78 and the anchor 15 are locked together during use.

The proximal pusher 78 may be coupled to the anchor 15 by an engagement wire 78a. The engagement wire 78a may run the length of the proximal pusher 78 from the connection point to the proximal end. A distal end of the engagement wire 78a may be threaded through a hole spanning at least one of the flat wires 53 and at least one of the spiral bands 17f when aligned in the delivery configuration and may act as an engagement pin to keep the wires and bands together during deployment of the anchor 15. One the anchor 15 is deployed, the proximal end of the engagement wire 78a may be translated proximally in order to disengage the engagement wire 78a from the anchor 15 and release the anchor 15 from the proximal pusher 78. The proximal end of the engagement wire 78a may be coupled to an actuation mechanism in/on the delivery device in order to translate the engagement wire 78a. Alternatively, the proximal end of the engagement wire 78a may be manually manipulated by, at, or near a proximal end of the delivery device in order to translate the engagement wire 78a. Once disengaged, the proximal pusher 78 may be retracted from the body, leaving the deployed anchor 15 adjacent the native valve as described herein.

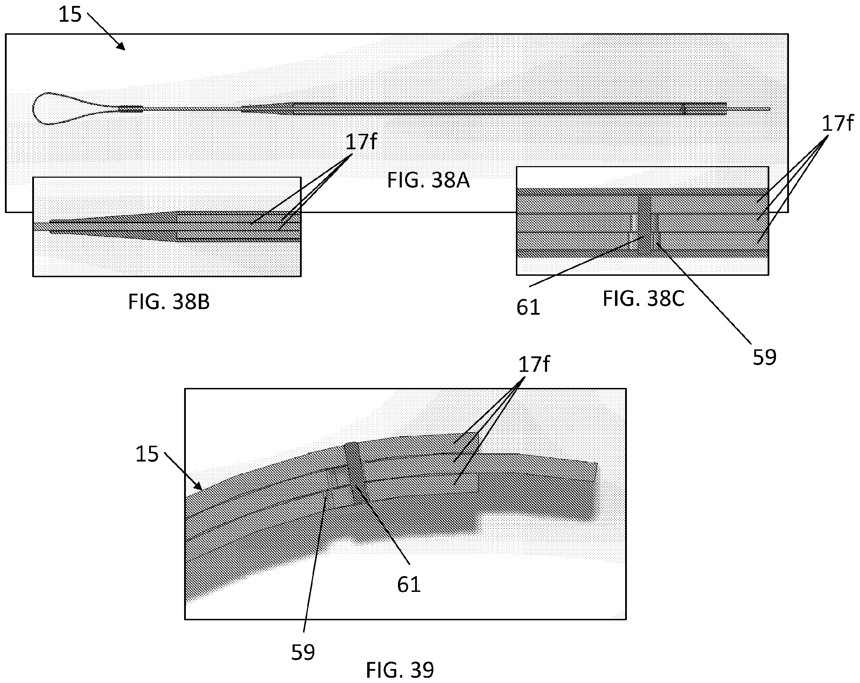

FIGS. 38A-39 show several views of a multi-ply anchor 15 comprising an optional stiffening or locking mechanism. FIGS. 38A-38C shows the anchor 15 in the undeployed (e.g., elongated) configuration. FIG. 39 shows the anchor 15 in the deployed (e.g., spiral) configuration. The multi-ply anchor 15 may be substantially similar to any of the multi-ply anchors described herein. In at least some instances, it may be beneficial to provide an anchor 15 with varying stiffness between the undeployed configuration and the deployed configuration. When in the undeployed configuration, increased flexibility may be desired in order to reduce strains and forces within the delivery device. In the deployed configuration, increased stiffness may be desired in order to ensure adequate rigidity of the anchor 15 for encircling the one or more structures of the native valve and the frame structure. Varying stiffness may be obtained by controlling the relative motion between the spiral bands 17f in the undeployed configuration and the deployed configuration. The anchor 15 may be configured such that the spiral bands 17f are allowed to freely slide relative to one another in the undeployed configuration but lock relative to one another in the deployed configuration.

The anchor 15 may comprise one or more stiffening or locking mechanisms configured to lock the spiral bands 17f relative to one another (e.g., to prevent movement of the spiral bands 17f and maintain the anchor 15 in the deployed configuration). The one or more locking mechanisms may be any of the locking mechanisms described herein or understood by one of ordinary skill in the art from the description herein. Locking the spiral bands 17f together may enable them to act as a larger meta-structure that is stiffer than the free-sliding bands.

As shown in Figures 38A-39, in some embodiments, the stiffening mechanism may comprise one or more slots 59 in the spiral bands 17f which correspond in shape to a pin 61 (or annular band) disposed adjacent a proximal end of the anchor 15. The pin-in-slot interface may be coupled such that the spiral bands 17f may have limited, clearly defined movement relative to one another depending on the size of the slots 59. In some embodiments, the one or more slots 59 may vary in length such that the anchor 15 is biased towards a particular (e.g., spiral, deployed) configuration when the spiral bands 17f are translated relative to one another. In this embodiment, the spiral bands 17f may be coupled (e.g., bonded) to one another and fixed at a distal end of the anchor 15 (i.e., so that the distal ends of the spiral bands 17f may be unable to translate relative to one another). In contrast, the proximal ends of the spiral bands 17f may comprise the slots 59 configured to enable translation of the spiral bands 17f relative to one another within the constraints of the pin-in-slot interface. The pin 61 may be free to move within the slots 59 of the spiral bands 17f (e.g., as the anchor 15 curves or spirals as shown in FIG. 39), but may prevent translation of the spiral bands 17f past a desired point, for example at a final radius of curvature of the deployed anchor 15. Engagement of the pin 61 with the slots 59 may lead the plurality of spiral bands 17f to behave approximately like a single element having a cross-section of the combined spiral bands 17f.

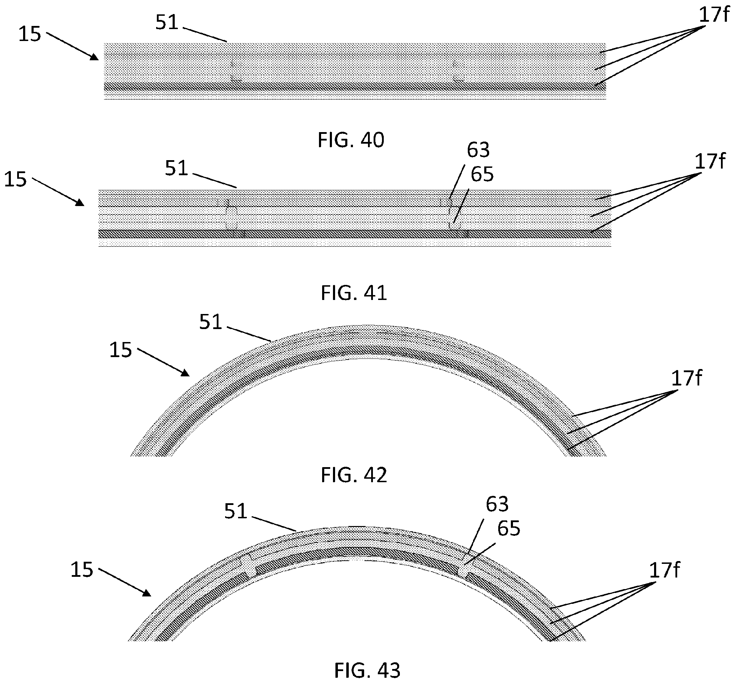

FIGS. 40-43 show several views of a multi-ply anchor 15 comprising another optional stiffening or locking mechanism. FIG. 40 shows a side view of the anchor 15 in the undeployed (e.g., elongated) configuration. FIG. 41 shows a side cross-sectional view of the anchor 15 in the undeployed configuration. FIG. 42 shows a side view of the anchor 15 in the deployed (e.g., spiral) configuration. FIG. 43 shows a side cross-sectional view of the anchor 15 in the deployed configuration. The multi-ply anchor 15 may be substantially similar to any of the multi-ply anchors described herein. The anchor 15 may be configured such that the spiral bands 17f are allowed to freely slide relative to one another in the undeployed configuration but lock relative to one another in the deployed configuration.

The anchor 15 may comprise one or more stiffening or locking mechanisms configured to lock the spiral bands 17f relative to one another and maintain the anchor 15 in the deployed configuration. The one or more locking mechanisms may be any of the locking mechanisms described herein or understood by one of ordinary skill in the art from the description herein. Locking the spiral bands 17f together may enable them to act as a larger meta-structure that is stiffer than the free-sliding bands.

In some embodiments, the stiffening mechanism may comprise one or more holes 63 in the spiral bands 17f which correspond in shape to a pin 65 or rivet disposed on an adjacent spiral band 17f. For example, a first spiral band 17f may comprise a first hole 63 and a second spiral band 17f may comprise a first pin 65 correspondingly-shaped to the first hole 63. The housing 51 of the anchor 15 may be configured to radially constrict the spiral bands 17f together. In the undeployed configuration (shown in FIGS. 40-41), the housing 51 may be loose and the pin(s) 65 may be unaligned with the hole(s) 63 such that the spiral bands 17f may move longitudinally relative to one another (which may create enhanced flexibility). In the deployed configuration (shown in FIGS. 42-43), the spiral bands 17f may bend into a pre-set (e.g., spiral) curvature, and the pin(s) 65 and hole(s) 63 may be aligned. The pins 65 may engage the holes 63, and the housing 51 may constrict to retain the engagement (which may create enhanced stiffness).

In some embodiments, the locking or stiffening mechanism may be a nonmechanical locking mechanism. For example, locking mechanism can be a friction or interference fit mechanism (e.g., if an inner spiral band 17f has a larger preset diameter or radius of curvature than an outer spiral band 17f that is positioned radially outwards of the inner spiral band 17f, then the inner spiral band 17f can push against the outer spiral band 17f and create an interference lock).

In some embodiments, the locking or stiffening mechanisms may lock all of the spiral bands 17f together. In other embodiments, the locking or stiffening mechanism my lock only a portion of the spiral bands 17f together and not all of the spiral bands 17f together.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the present disclosure. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present disclosure.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The foregoing descriptions of specific embodiments of the present disclosure have been presented for purposes of illustration and description. They are not intended to be exhaustive or to limit the invention to the precise forms disclosed, and obviously many modifications and variations are possible in light of the above teaching. The embodiments were chosen and described to best explain the principles of the invention and its practical application, to thereby enable others skilled in the art to best utilize the present disclosure and various embodiments with various modifications as are suited to the particular use contemplated. It is intended that the scope of the invention be defined by the Claims appended hereto.

While preferred embodiments of the present disclosure have been shown and described herein, it will be obvious to those skilled in the art that such embodiments are provided by way of example only. It is intended that the following claims define the scope of the invention.

## Claims

1. A system for treating a diseased native heart valve in a patient, the system comprising:
a frame structure (12) having an unexpanded configuration and an expanded configuration; and
an anchor (15) comprising a free end (22) and having a flat spiral shape, wherein the anchor comprises housing (51), a lumen (71) disposed within the housing, and a plurality of spiral bands (17f) translatably disposed within the lumen;
wherein the frame structure is configured to be actuated from the unexpanded configuration to the expanded configuration adjacent a native heart valve (4) in a patient;
wherein the anchor is configured to anchor the frame structure to the native heart valve when the frame structure is in the expanded configuration adjacent the native heart valve; and
wherein the plurality of spiral bands (17f) are slidably disposed within the lumen (71) relative to one another.

2. The system of claim 1, wherein the anchor (15) comprises a delivery configuration and a deployed configuration.

3. The system of claim 2, wherein the anchor comprises the flat spiral shape in the deployed configuration.

4. The system of claim 2, wherein the anchor (15) comprises one or more locking mechanisms configured to maintain the anchor in the deployed configuration.

5. The system of claim 4, wherein the one or more locking mechanisms comprise a frictional band (21), a polymer coating, or one or more key and one or more key hole features (19).

6. The system of claim 4, wherein the one or more locking mechanisms comprise a pin (65) on a first spiral band of the plurality of spiral bands (17f) and a correspondingly-shaped hole (63) on a second spiral band of the plurality of spiral bands.

7. The system of claim 4, wherein the one or more locking mechanisms comprise a plurality of slots (59) on the plurality of spiral bands (17f) and a correspondingly-shaped pin (61).

8. The system of claim 4, wherein the one or more locking mechanisms comprise a first feature (24) disposed on a first loop of the anchor (15) and a correspondingly-shaped second feature (24) disposed on a second loop of the anchor, the first and second features configured to nest with one another when the anchor is in the deployed configuration.

9. The system of claim 2, further comprising a delivery device (30), the delivery device comprising an outer sheath, wherein the anchor (15) is disposed in a lumen of the outer sheath and maintained in the delivery configuration by radial constriction from the outer sheath, and wherein advancement of the anchor out of the lumen of the outer sheath actuates the anchor into the deployed configuration.

10. The system of claim 9, wherein the delivery device (30) further comprises an inner shaft (52), wherein the inner shaft is disposed within the lumen of the outer sheath.

11. The system of claim 10, wherein a proximal portion of the frame structure (12) or a proximal end of the anchor (15) is coupled to a distal portion of the inner shaft (52).

12. The system of claim 10, wherein advancement of the inner shaft (52) towards an opening of the lumen of the outer sheath advances the anchor (15) out of the lumen of the outer shaft and actuates the anchor into the deployed configuration.

13. The system of claim 9, wherein the frame structure (12) is detachably coupled to the delivery device (30) in the unexpanded configuration during delivery to the native heart valve (4).

14. The system of claim 9, wherein expansion of the frame structure (12) to the expanded configuration detaches the frame structure from the delivery device (30).

15. The system of claim 1, wherein the expanded configuration of the frame structure (12) is a generally tubular expanded shape.

## Patentansprüche

1. System zum Behandeln einer erkrankten nativen Herzklappe in einem Patienten, wobei das System umfasst:
eine Rahmenstruktur (12) mit einer nicht ausgedehnten Konfiguration und einer ausgedehnten Konfiguration; und
einen Anker (15), der ein freies Ende (22) umfasst und eine flache Spiralform aufweist, wobei der Anker ein Gehäuse (51), ein Lumen (71), das innerhalb des Gehäuses angeordnet ist, und eine Mehrzahl von Spiralbändern (17f) umfasst, die innerhalb des Lumens translatierbar angeordnet sind;
wobei die Rahmenstruktur dazu konfiguriert ist, aus der nicht ausgedehnten Konfiguration in die ausgedehnte Konfiguration benachbart zu einer nativen Herzklappe (4) in einem Patienten betätigt zu werden;
wobei der Anker dazu konfiguriert ist, die Rahmenstruktur an der nativen Herzklappe zu verankern, wenn sich die Rahmenstruktur in der ausgedehnten Konfiguration benachbart zu der nativen Herzklappe befindet; und
wobei die Mehrzahl von Spiralbändern (17f) innerhalb des Lumens (71) relativ zueinander verschiebbar angeordnet ist.

2. System nach Anspruch 1, wobei der Anker (15) eine Abgabekonfiguration und eine entfaltete Konfiguration umfasst.

3. System nach Anspruch 2, wobei der Anker in der entfalteten Konfiguration die flache Spiralform umfasst.

4. System nach Anspruch 2, wobei der Anker (15) einen oder mehrere Verriegelungsmechanismen umfasst, die dazu konfiguriert sind, den Anker in der entfalteten Konfiguration zu halten.

5. System nach Anspruch 4, wobei der eine oder die mehreren Verriegelungsmechanismen ein Reibungsband (21), eine Polymerbeschichtung oder ein oder mehrere Schlüssel- und ein oder mehrere Schlüssellochmerkmale (19) umfassen.

6. System nach Anspruch 4, wobei der eine oder die mehreren Verriegelungsmechanismen einen Stift (65) an einem ersten Spiralband der Mehrzahl von Spiralbändern (17f) und ein entsprechend geformtes Loch (63) an einem zweiten Spiralband der Mehrzahl von Spiralbändern umfassen.

7. System nach Anspruch 4, wobei der eine oder die mehreren Verriegelungsmechanismen eine Mehrzahl von Schlitzen (59) an der Mehrzahl von Spiralbändern (17f) und einen entsprechend geformten Stift (61) umfassen.

8. System nach Anspruch 4, wobei der eine oder die mehreren Verriegelungsmechanismen ein erstes Merkmal (24), das an einer ersten Schleife des Ankers (15) angeordnet ist, und ein entsprechend geformtes zweites Merkmal (24) umfassen, das an einer zweiten Schleife des Ankers angeordnet ist, wobei das erste und das zweite Merkmal dazu konfiguriert sind, ineinander zu passen, wenn sich der Anker in der entfalteten Konfiguration befindet.

9. System nach Anspruch 2, ferner umfassend eine Abgabevorrichtung (30), wobei die Abgabevorrichtung eine äußere Hülle umfasst, wobei der Anker (15) in einem Lumen der äußeren Hülle angeordnet ist und durch radiale Verengung durch die äußere Hülle in der Abgabekonfiguration gehalten wird, und wobei ein Vorschieben des Ankers aus dem Lumen der äußeren Hülle den Anker in die entfaltete Konfiguration betätigt.

10. System nach Anspruch 9, wobei die Abgabevorrichtung (30) ferner einen inneren Schaft (52) umfasst, wobei der innere Schaft innerhalb des Lumens der äußeren Hülle angeordnet ist.

11. System nach Anspruch 10, wobei ein proximaler Abschnitt der Rahmenstruktur (12) oder ein proximales Ende des Ankers (15) mit einem distalen Abschnitt des inneren Schafts (52) gekoppelt ist.

12. System nach Anspruch 10, wobei das Vorschieben des inneren Schafts (52) in Richtung einer Öffnung des Lumens der äußeren Hülle den Anker (15) aus dem Lumen des äußeren Schafts vorschiebt und den Anker in die entfaltete Konfiguration betätigt.

13. System nach Anspruch 9, wobei die Rahmenstruktur (12) in der nicht ausgedehnten Konfiguration während der Abgabe an die native Herzklappe (4) lösbar mit der Abgabevorrichtung (30) gekoppelt ist.

14. System nach Anspruch 9, wobei die Ausdehnung der Rahmenstruktur (12) in die ausgedehnte Konfiguration die Rahmenstruktur von der Abgabevorrichtung (30) löst.

15. System nach Anspruch 1, wobei die ausgedehnte Konfiguration der Rahmenstruktur (12) eine im Allgemeinen röhrenförmige ausgedehnte Form aufweist.

## Revendications

1. Système de traitement d'une valve cardiaque native malade chez un patient, le système comprenant :
une structure de base (12) présentant une configuration non étendue et une configuration étendue, et
un ancrage (15) comprenant une extrémité libre (22) et présentant une forme en spirale plate, l'ancrage comprenant une enveloppe (51), une lumière (71) ménagée à l'intérieur de l'enveloppe, et une pluralité de bandes en spirales (17f) disposées avec faculté de translation à l'intérieur de la lumière ;
ladite structure de base étant conçue pour passer de la configuration non étendue à la configuration étendue à proximité d'une valve cardiaque native (4) d'un patient,
ledit ancrage étant conçu pour ancrer la structure de base à la valve cardiaque native lorsque la structure de base est en configuration étendue à proximité de la valve cardiaque native, et
ladite pluralité de bandes en spirales (17f) étant disposées coulissantes à l'intérieur de la lumière (71) les unes par rapport aux autres.

2. Système selon la revendication 1, dans lequel l'ancrage (15) comprend une configuration de mise en place et une configuration déployée.

3. Système selon la revendication 2, dans lequel l'ancrage comprend ladite forme en spirale plate en configuration déployée.

4. Système selon la revendication 2, dans lequel l'ancrage (15) comprend un ou plusieurs mécanismes de verrouillage conçus pour maintenir l'ancrage en configuration déployée.

5. Système selon la revendication 4, dans lequel le ou les mécanismes de verrouillage comprennent une bande de frottement (21), un revêtement polymère, ou un ou plusieurs systèmes à clavettes et alésages (19).

6. Système selon la revendication 4, dans lequel le ou les mécanismes de verrouillage comprennent un ergot (65) sur une première bande en spirale de la pluralité de bandes en spirale (17f) et un trou (63) de forme correspondante sur une deuxième bande en spirale de la pluralité de bandes en spirale.

7. Système selon la revendication 4, dans lequel le ou les mécanismes de verrouillage comprennent une pluralité de fentes (59) sur la pluralité de bandes en spirale (17f) et une partie mâle de forme correspondante (61).

8. Système selon la revendication 4, dans lequel le ou les mécanismes de verrouillage comprennent un premier élément (24) disposé sur une première boucle de l'ancrage (15) et un deuxième élément (24) de forme correspondante disposé sur une deuxième boucle de l'ancrage, les premier et deuxième éléments étant conçus pour s'emboîter l'un dans l'autre lorsque l'ancrage est en configuration déployée.

9. Système selon la revendication 2, comprenant en outre un dispositif de mise en place (30), le dispositif de mise en place comprenant une gaine extérieure, ledit ancrage (15) étant disposé dans une lumière de la gaine extérieure et maintenu en configuration de mise en place par une constriction radiale de la gaine extérieure, et l'avancée de l'ancrage hors de la lumière de la gaine extérieure faisant passer l'ancrage en configuration déployée.

10. Système selon la revendication 9, dans lequel le dispositif de mise en place (30) comprend en outre une tige intérieure (52), la tige intérieure étant disposée à l'intérieur de la lumière de la gaine extérieure.

11. Système selon la revendication 10, dans lequel une portion proximale de la structure de base (12) ou une extrémité proximale de l'ancrage (15) est couplée à une portion distale de la tige intérieure (52).

12. Système selon la revendication 10, dans lequel l'avancée de la tige intérieure (52) vers une ouverture de la lumière de la gaine extérieure entraîne une avancée de l'ancrage (15) hors de la lumière de la tige extérieure et fait passer l'ancrage en la configuration déployée.

13. Système selon la revendication 9, dans lequel la structure de base (12) est couplée de manière amovible au dispositif de mise en place (30) en configuration non étendue pendant la mise en place dans la valve cardiaque native (4).

14. Système selon la revendication 9, dans lequel le passage de la structure de base (12) vers la configuration étendue détache la structure de base du dispositif de mise en place (30).

15. Système selon la revendication 1, dans lequel la configuration étendue de la structure de base (12) est une forme étendue globalement tubulaire.
